(19) Europäisches Patentamt
European Patent Office
Office européen des brevets

(11) EP 4 151 220 A1

(12) # EUROPEAN PATENT APPLICATION
published in accordance with Art. 153(4) EPC

(43) Date of publication:
**22.03.2023 Bulletin 2023/12**

(21) Application number: **21804169.7**

(22) Date of filing: **14.05.2021**

(51) International Patent Classification (IPC):
***A61K 31/5365*** *(2006.01)* ***A61K 38/21*** *(2006.01)*
***A61K 31/52*** *(2006.01)* ***A61K 31/522*** *(2006.01)*
***A61P 31/20*** *(2006.01)*

(52) Cooperative Patent Classification (CPC):
**A61K 31/52; A61K 31/522; A61K 31/5365; A61K 38/21; A61P 31/20**

(86) International application number:
**PCT/CN2021/093805**

(87) International publication number:
**WO 2021/228222 (18.11.2021 Gazette 2021/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.05.2020 CN 202010412771**

(71) Applicant: **Fujian Akeylink Biotechnology Co., Ltd.**
**Ningde, Fujian 355300 (CN)**

(72) Inventors:
- **CAI, Zhe**
  **Shanghai 200131 (CN)**
- **SUN, Fei**
  **Shanghai 200131 (CN)**
- **HU, Yanbin**
  **Shanghai 200131 (CN)**
- **DING, Charles Z.**
  **Shanghai 200131 (CN)**
- **CHEN, Shuhui**
  **Shanghai 200131 (CN)**
- **WU, Wenqiang**
  **Ningde, Fujian 355300 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **COMBINATION FOR TREATING HEPATITIS B**

(57) The present invention relates to a combination of a compound represented by formula (I) and other drugs for treating hepatitis B that is used for treating hepatitis B, and use of the combination in the preparation of drugs for treating hepatitis B.

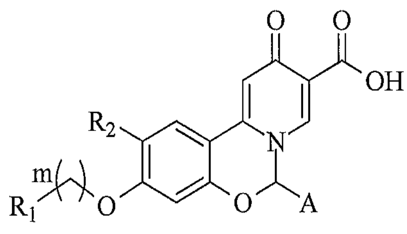

(I)

EP 4 151 220 A1

## Description

The present application claims the right of the following priority of Chinese patent application CN202010412771.7 filed on May 15, 2020. The contents of the above Chinese patent application are incorporated herein by reference in its entireties.

TECHNICAL FIELD

The present disclosure belongs to the biomedical field, and relates to a class of combinations of a compound represented by formula (I) for the treatment of hepatitis B and other medicaments for the treatment of hepatitis B, and use of the combination in the manufacture of a medicament for the treatment of hepatitis B.

BACKGROUND

Viral hepatitis B, abbreviated as hepatitis B, is a disease caused by Hepatitis B Virus (HBV) infection in the body. Hepatitis B virus is a hepatotropic virus that mainly exists in hepatocytes and damages hepatocytes, causing inflammation, necrosis and fibrosis of hepatocytes. Viral hepatitis B is divided into two types, acute hepatitis B and chronic hepatitis B. Most adults with acute hepatitis B can heal themselves through their inherent immune function. However, chronic hepatitis B (CHB) has become a major challenge for global health care, and a major cause of chronic liver disease, cirrhosis and hepatocellular carcinoma (HCC). It is estimated that 2 billion people worldwide are infected with chronic hepatitis B virus, and more than 350 million people have progressed to hepatitis B. Nearly 600, 000 people die each year from the complications of chronic hepatitis B. China is a high-prevalence area of hepatitis B, and accumulated a lot of patients with hepatitis B, which is a serious hazard. According to the data, there are about 93 million people infected with hepatitis B virus in China, and about 20 million of them are diagnosed with chronic hepatitis B, among them, 10%-20% can progress to cirrhosis, and 1%-5% can progress to HCC.

The key to functional cure for hepatitis B is to clear HBsAg (hepatitis B virus surface antigen) and produce surface antibodies. Quantification of HBsAg is a very important biological indicator. In chronically infected patients, a decrease in HBsAg and seroconversion are rarely observed, which is the end point of current treatment.

The surface antigen protein of hepatitis B virus (HBV) plays a very important role in the process of HBV invasion into liver cells, and is of great significance for the prevention and treatment of HBV infection. Surface antigen proteins include large (L), medium (M) and small (S) surface antigen proteins that share a common C-terminal S region. They are expressed in an open reading frame, the different lengths of which are determined by the three AUG initiation codons of the reading frame, the different lengths of which are determined by the three AUG start codons of the reading frame. These three surface antigen proteins include pre-S1 /pre- S2/S, pre-S2/S and S domains. The HBV surface antigen protein is integrated into the endoplasmic reticulum (ER) membrane and initiated by the N-terminal signal sequence. They not only constitute the basic structure of virions, but also form globular and filamentous subviral particles (SVPs, HBsAg), aggregated in ER, host ER and pre-Golgi apparatus, SVP contains mostly S Surface antigen protein. The L protein is critical in the aspects of morphogenesis of the virus and the interaction of the nucleocapsid, but is not necessary for the formation of SVP. Because of their lack of nucleocapsids, the SVPs are non-infectious. SVPs are heavily involved in disease progression, especially in immune response to hepatitis B virus, in the blood of infected people, the content of SVPs is at least 10, 000 times the number of viruses, and it traps the immune system and weakens the body's immune response to hepatitis B virus. HBsAg also inhibits human innate immunity, inhibits the production of cytokines induced by polysaccharides (LPS) and IL-2, inhibits DC function of dendritic cells, and inhibits the induced activity of LPS against the interference kinase -1/2 in ERK-1/2 and c-Jun N-terminal in monocytes. It is worth noting that disease progression of cirrhosis and hepatocellular carcinoma is also largely associated with persistent secretion of HBsAg. These researches suggest that HBsAg plays an important role in the progress of chronic hepatitis.

The currently approved anti-HBV drugs are mainly immunomodulators (interferon-$\alpha$ and peginterferon-$\alpha$-2$\alpha$) and antiviral therapeutic drugs (Lamivudine, Adefovir Disoproxil, Entecavir, Telbivudine, Tenofovir, Clevudine, etc.). Among them, antiviral therapeutic drugs belong to nucleoside or nucleotide drugs, and their mechanism of action is to inhibit the synthesis of HBV DNA, instead of directly reducing HBsAg levels. As the prolonged treatment, nucleotide drugs have shown HBsAg clearance rate which is similar to natural observations

Clinically available therapies exhibit a poor efficacy in reducing HBsAg, therefore, the development of small molecule oral inhibitors that can effectively reduce HBsAg is currently required for clinical use.

Even though WO2016128335A1 has disclosed a series of 2-oxo-6,7-dihydrobenzo[*a*]quinazine-3-carboxylic acid derivatives for the treatment or prevention of hepatitis B virus infection, this series of fused ring compounds still have the problems of strong molecular rigidity, insufficient solubility, and easy aromatization. Therefore, for clinical applications, the demand for drugs with better druggability still exists.

CONTENT OF THE PRESENT INVENTION

In one aspect, the present disclosure provides a combination, comprising a compound represented by formula (I) and other medicaments for the treatment of hepatitis B.

In another aspect, the present disclosure provides a composition, comprising the combination of the present disclosure and a pharmaceutically acceptable carrier and/or excipient.

In another aspect, the present disclosure provides a kit, comprising the composition of the present disclosure.

In another aspect, the present disclosure provides a use of the combination, the composition or the kit in the manufacture of a medicament for the treatment of hepatitis B. The present disclosure also provides a method for the treatment of hepatitis B, comprising administering an effective amount of the combination, the composition or the kit of the present disclosure into the subject. The present disclosure also provides the combination, the composition or the kit of the present disclosure for use in the treatment of hepatitis B.

In another aspect, the present disclosure provides a use of the compound represented by formula (I) in combination with other medicaments for the treatment of hepatitis B in the manufacture of a medicament for the treatment of hepatitis B. The present disclosure also provides a method for the treatment of hepatitis B, comprising administering an effective amount of the compound represented by formula (I) and other medicaments for the treatment of hepatitis B into the subject. The present disclosure also provides the compound represented by formula (I) in combination with other medicaments for the treatment of hepatitis B for use in the treatment of hepatitis B.

The present disclosure provides a combination, comprising a compound represented by formula (I) or a pharmaceutically acceptable salt thereof,

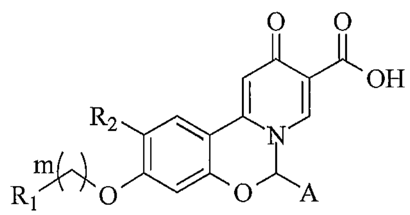

(I)

wherein,

$R_1$ is selected from H, OH, CN, $NH_2$, $C_{1-5}$ alkyl, $C_{1-5}$ heteroalkyl, $C_{2-5}$ alkynyl, $C_{3-6}$ cycloalkyl and 3- to 6-membered heterocycloalkyl, and the $C_{1-5}$ alkyl, $C_{1-5}$ heteroalkyl, $C_{2-5}$ alkynyl, $C_{3-6}$ cycloalkyl and 3- to 6-membered heterocycloalkyl are optionally substituted by 1, 2 or 3 R;

$R_2$ is selected from H, halogen, $C_{1-3}$ alkyl and $C_{1-3}$ heteroalkyl, and the $C_{1-3}$ alkyl and $C_{1-3}$ heteroalkyl are optionally substituted by 1, 2 or 3 R;

m is selected from 0, 1, 2, 3, 4 and 5;

A is selected from phenyl and 5- to 6-membered heteroaryl, and the phenyl and 5- to 6-membered heteroaryl are optionally substituted by 1, 2 or 3 R;

R is selected from H, halogen, OH, CN, $NH_2$, =O, $CH_3$, $CH_3CH_2$, $CH_3O$, $CF_3$, $CHF_2$, $CH_2F$;

the $C_{1-5}$ heteroalkyl, 3- to 6-membered heterocycloalkyl, $C_{1-3}$ heteroalkyl and 5- to 6-membered heteroaryl each independently comprise 1, 2 or 3 heteroatoms or heteroatom groups each independently selected from N, -O-, =O, -S-, -NH-, -(C=O)-, -(S=O)- and $-(S=O)_2-$,

which is characterized by further comprising an immunomodulator.

In some embodiments of the present disclosure, the combination comprises the compound represented by formula (I) or the pharmaceutically acceptable salt thereof and the immunomodulator, which is characterized in that, the immunomodulator is selected from interferon, preferably peginterferon $\alpha$-2a or interferon $\alpha$-2b (IFN$\alpha$-2b).

In some embodiments of the present disclosure, the combination comprises the compound represented by formula (I) or the pharmaceutically acceptable salt thereof and the immunomodulator, which is characterized by further comprising a nucleotide reverse transcriptase inhibitor.

In some embodiments of the present disclosure, the combination comprises the compound represented by formula (I) or the pharmaceutically acceptable salt thereof, the immunomodulator and the nucleotide reverse transcriptase inhibitor, which is characterized in that, the nucleotide reverse transcriptase inhibitor is selected from tenofovir disoproxil fumarate and tenofovir alafenamide fumarate.

In some embodiments of the present disclosure, the combination comprises the compound represented by

formula (I) or the pharmaceutically acceptable salt thereof and the immunomodulator, and the nucleoside reverse transcriptase inhibitor, which is characterized in that, the immunomodulator is selected from interferon, preferably peginterferon $\alpha$-2a or interferon $\alpha$-2b, and the nucleotide reverse transcriptase inhibitor is selected from tenofovir disoproxil fumarate and tenofovir alafenamide fumarate.

The present disclosure also provides a combination, comprising the compound represented by formula (I) or the pharmaceutically acceptable salt thereof, which is characterized by further comprising a nucleotide reverse transcriptase inhibitor or a nucleoside reverse transcriptase inhibitor.

In some embodiments of the present disclosure, the combination comprises the compound represented by formula (I) or the pharmaceutically acceptable salt thereof and the nucleotide reverse transcriptase inhibitor, which is characterized in that, the nucleotide reverse transcriptase inhibitor is selected from tenofovir disoproxil fumarate and tenofovir alafenamide fumarate.

In some embodiments of the present disclosure, the combination comprises the compound represented by formula (I) or the pharmaceutically acceptable salt thereof and the nucleoside reverse transcriptase inhibitor, which is characterized in that, the nucleoside reverse transcriptase inhibitor is selected from Entecavir.

In some embodiments of the present disclosure, in the combination, R is selected from H, F, Cl, Br, OH, $CH_3$, $CH_3O$, $CF_3$, $CHF_2$, $CH_2F$, and the other variables are as defined herein.

In some embodiments of the present disclosure, in the combination, $R_1$ is selected from H, OH, CN, $NH_2$, $CH_3$, $CH_3CH_2$, $CH_3CH_2CH_2$, $CH_3CH_2CH_2CH_2$, $CH_3O$, $CH_3CH_2O$, $CH_3S$, $CH_3S(=O)$, $CH_3S(=O)_2$, $CH_3SCH_2$, $CH_3CH_2S$, $CH_3NH$,

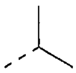, 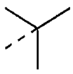, 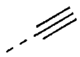,

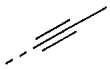, 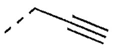, 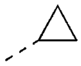, 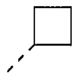, 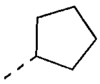, 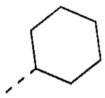,

pyrrolidinyl, piperidinyl, tetrahydropyranyl, morpholinyl, 2-pyrrolidonyl and 3-pyrrolidonyl, and the $CH_3$, $CH_3CH_2$, $CH_3CH_2CH_2$, $CH_3CH_2CH_2CH_2$, $CH_3O$, $CH_3CH_2O$, $CH_3S$, $CH_3S(=O)$, $CH_3S(=O)_2$, $CH_3SCH_2$, $CH_3CH_2S$, $CH_3NH$,

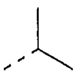, 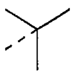, 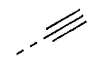, 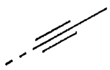, 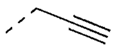, 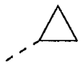, 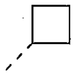, 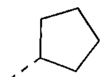,

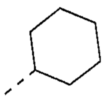,

pyrrolidinyl, piperidinyl, tetrahydropyranyl, morpholinyl, 2-pyrrolidonyl and 3-pyrrolidonyl are optionally substituted by 1, 2 or 3 R, and the other variables are as defined herein.

In some embodiments of the present disclosure, in the combination, $R_1$ is selected from H, OH, CN, $NH_2$, $CH_3$, $CH_3CH_2$, $CH_3CH_2CH_2$, $CH_3CH_2CH_2CH_2$, $CH_3O$, $CH_3CH_2O$, $CH_3S$, $CH_3S(=O)$, $CH_3S(=O)_2$, $CH_3SCH_2$, $CH_3SCH_2S$, $CH_3NH$,

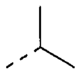, 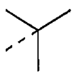, 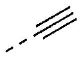,

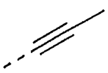, 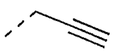, 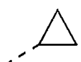, 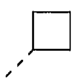, 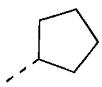, 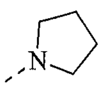, 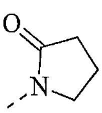, 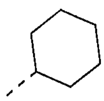, 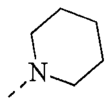,

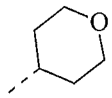 and 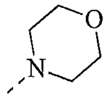,

and the $CH_3$, $CH_3CH_2$, $CH_3CH_2CH_2$, $CH_3CH_2CH_2CH_2$, $CH_3O$, $CH_3CH_2O$, $CH_3S$, $CH_3S(=O)$, $CH_3S(=O)_2$, $CH_3SCH_2$, $CH_3CH_2S$, $CH_3NH$,

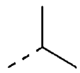, 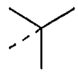,

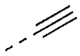, 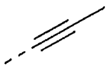, 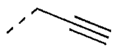, 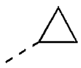, 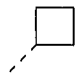, 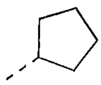, 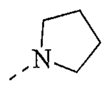, 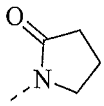, 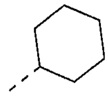, 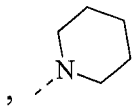,

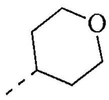 and 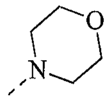

are optionally substituted by 1, 2 or 3 R, and the other variables are as defined herein.

In some embodiments of the present disclosure, in the combination, $R_1$ is selected from H, OH, $CH_3$, $CHF_2$, $CH_3O$,

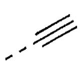, 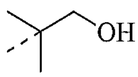, 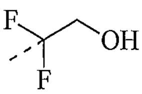, 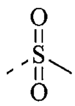, 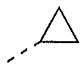, 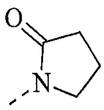

and

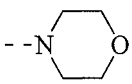,

-and the other variables are as defined herein.

In some embodiments of the present disclosure, in the combination, $R_2$ is selected from H, F, Cl, Br, $CH_3$, $CH_3CH_2$, $CH_3O$, $CH_3CH_2O$ and

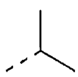,

and the $CH_3$, $CH_3CH_2$, $CH_3O$, $CH_3CH_2O$ and

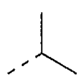

are optionally substituted by 1, 2 or 3 R, and the other variables are as defined herein.

In some embodiments of the present disclosure, in the combination, $R_2$ is selected from Cl and $CH_3O$, and the other variables are as defined herein.

In some embodiments of the present disclosure, in the combination, A is selected from: phenyl, thienyl, thiazolyl, isothiazolyl, oxazolyl and isoxazolyl, each of which is optionally substituted by 1, 2 or 3 R, and the other variables are as defined herein.

In some embodiments of the present disclosure, in the combination, A is selected from:

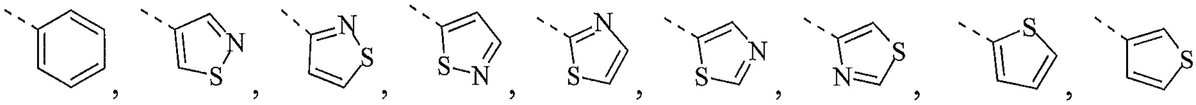

and

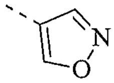,

each of which is optionally substituted by 1, 2 or 3 R, and the other variables are as defined herein.

In some embodiments of the present disclosure, in the combination, A is selected from:

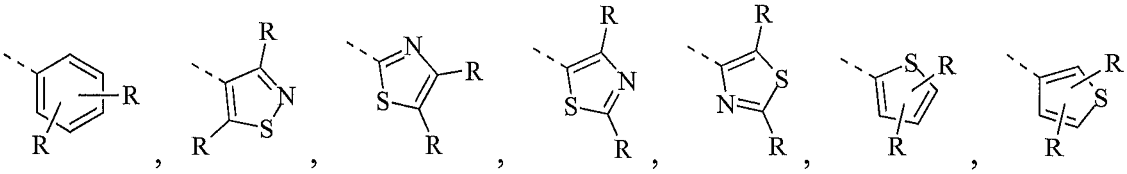

and

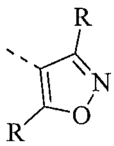,

and the other variables are as defined herein.

In some embodiments of the present disclosure, in the combination, A is selected from:

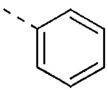, 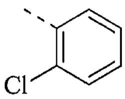, 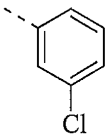, 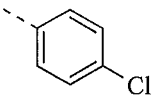, 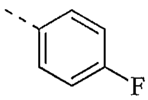, 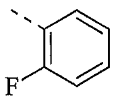, 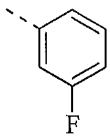, 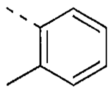,

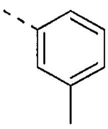, 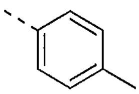, 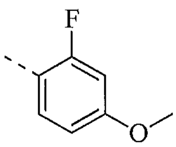, 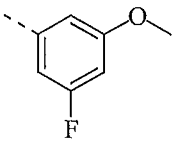, 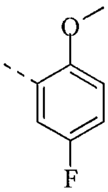, 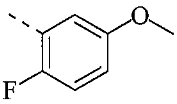, 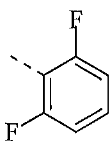,

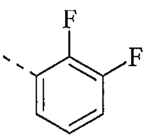, 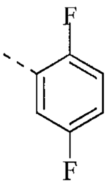, 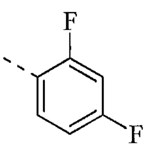, 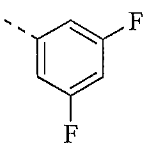, 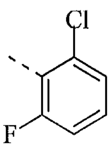, 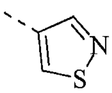, 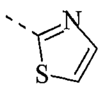, 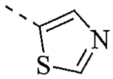,

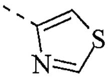, 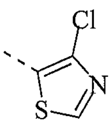, 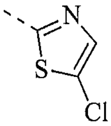, 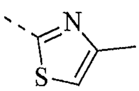, 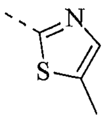, 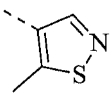, 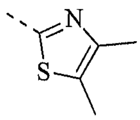, 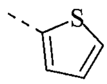,

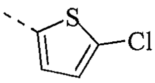, 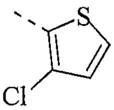, 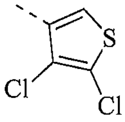 and 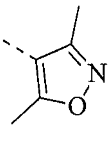

, and the other variables are as defined herein.

In some embodiments of the present disclosure, in the combination, m is 3, and the other variables are as defined herein.

In some embodiments of the present disclosure, in the combination, $R_2$ is selected from Cl and $CH_3O$, and the other variables are as defined herein.

In some embodiments of the present disclosure, in the combination, $R_1$ is $CH_3O$, and the other variables are as defined herein.

In some embodiments of the present disclosure, in the combination, m is 1, and the other variables are as defined herein.

In some embodiments of the present disclosure, in the combination, $R_2$ is Cl, and the other variables are as defined herein.

In some embodiments of the present disclosure, in the combination, $R_1$ is

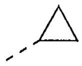,

and the other variables are as defined herein.

In some embodiments of the present disclosure, in the combination, A is selected from:

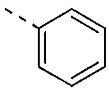, 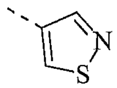, 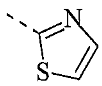, 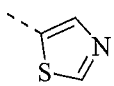, 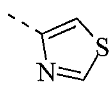, 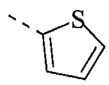 and 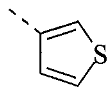,

each of which is optionally substituted by 1, 2 or 3 R, and the other variables are as defined herein.

In some embodiments of the present disclosure, in the combination, the compound is selected from:

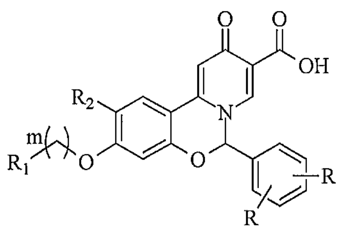

(II),

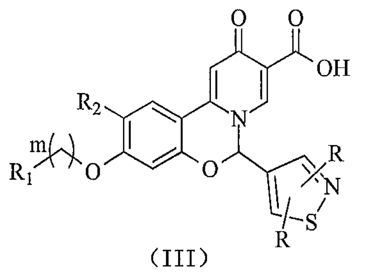

(III),

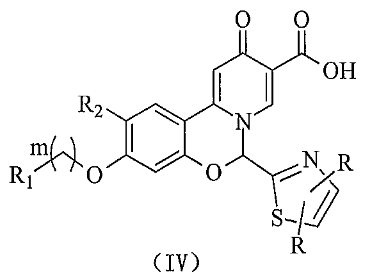

(IV),

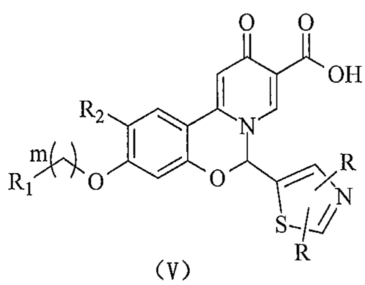

(V),

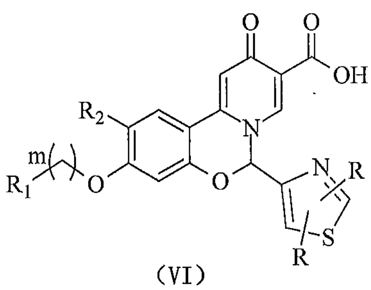

(VI),

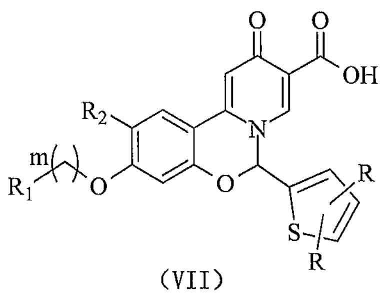

(VII),

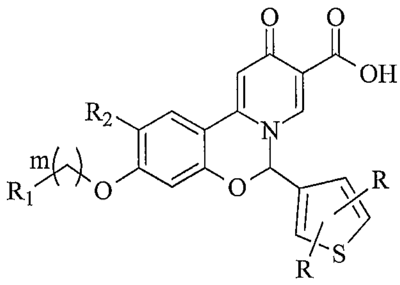

(VIII) ,

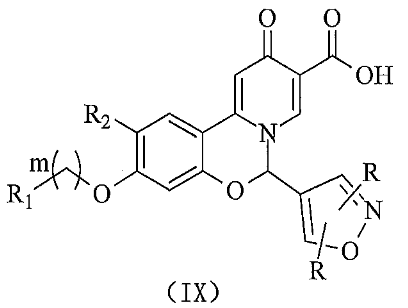

(IX) ,

wherein,

$R_1$, $R_2$, R and m are as defined above.

In some embodiments of the present disclosure, in the combination, the compound is selected from:

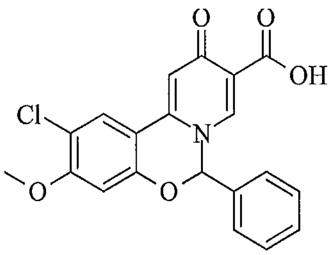

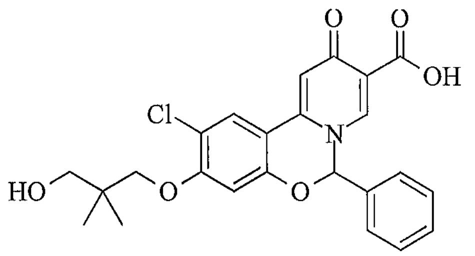

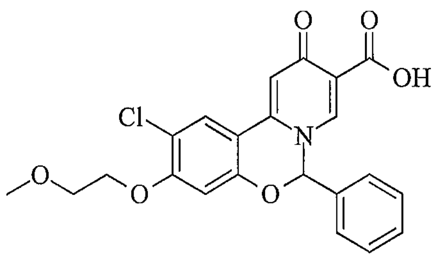

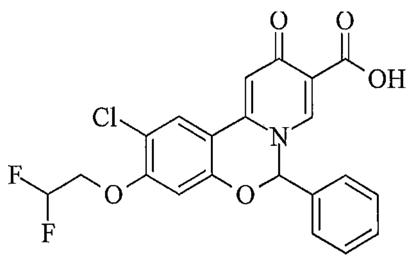

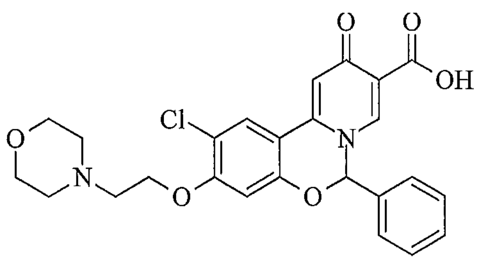

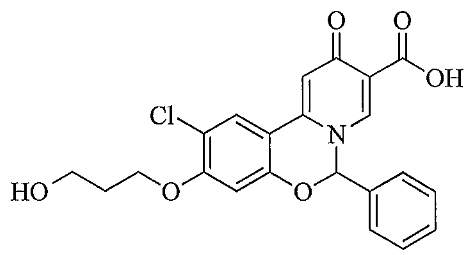

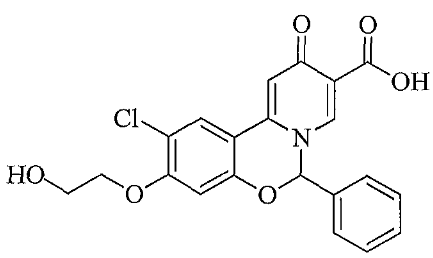

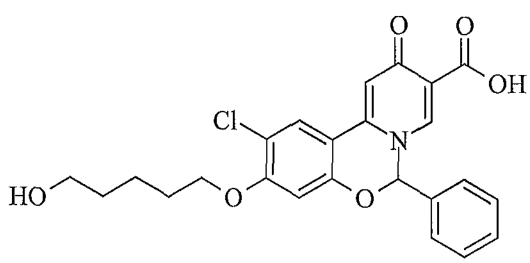

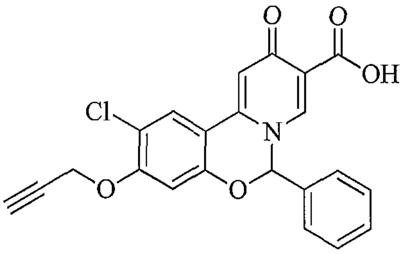

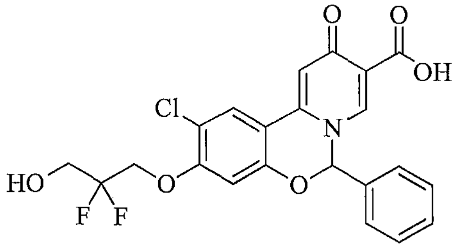

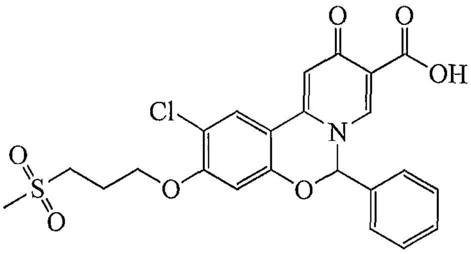

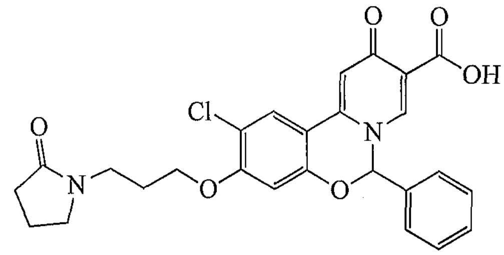

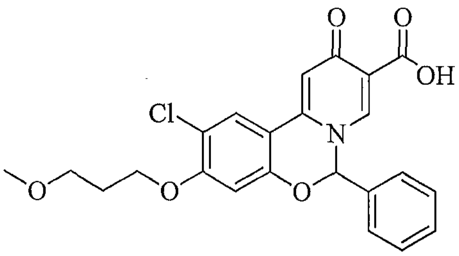

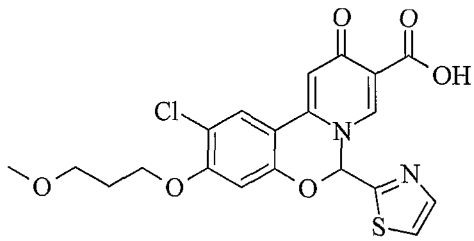

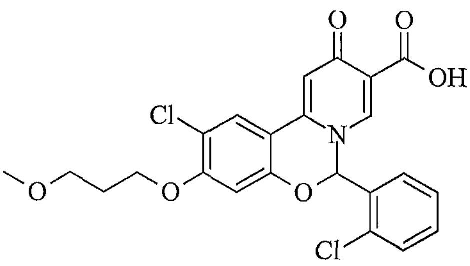

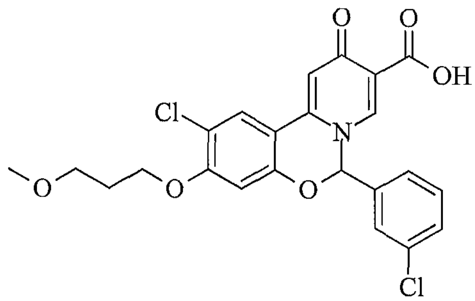

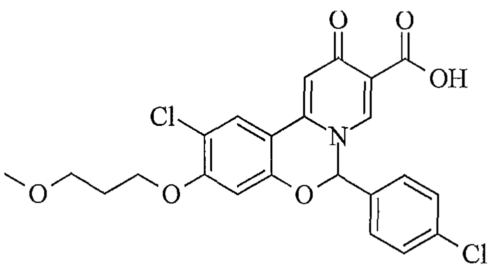

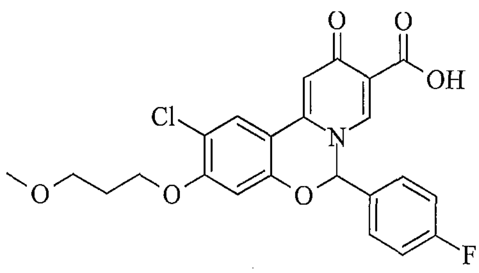

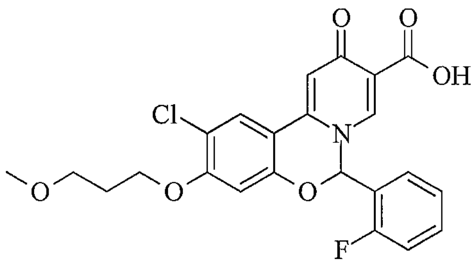

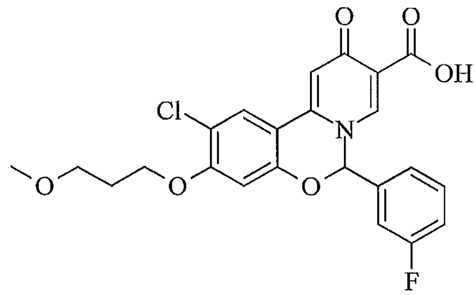

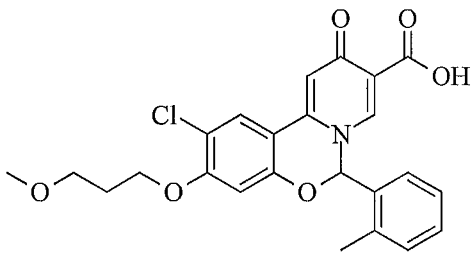

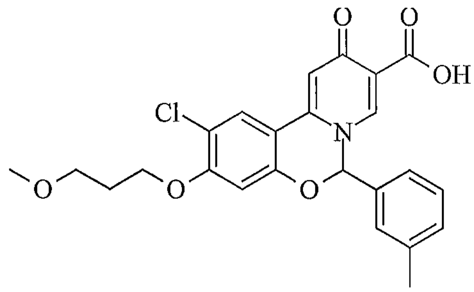

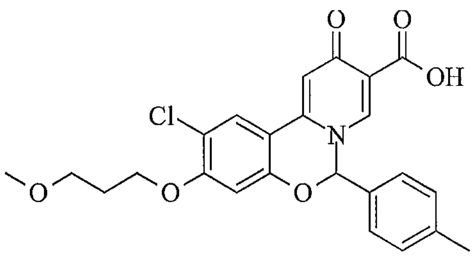

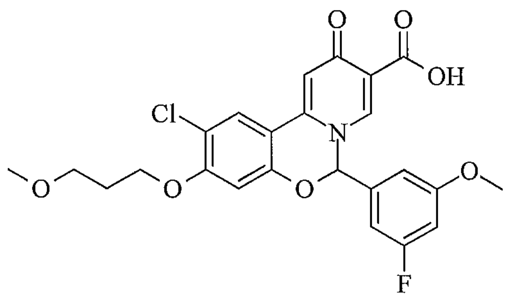

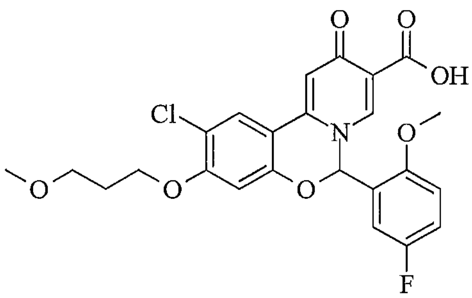

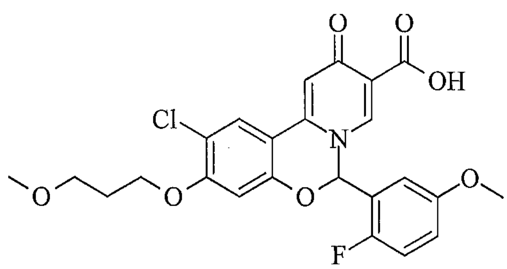

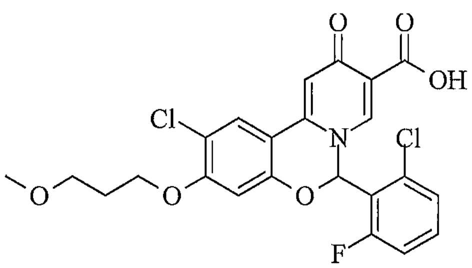

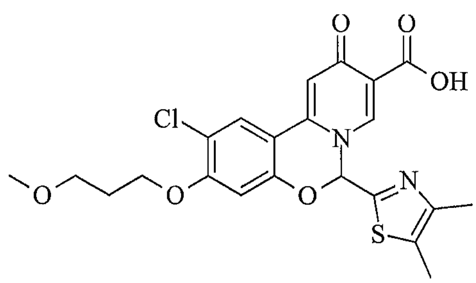

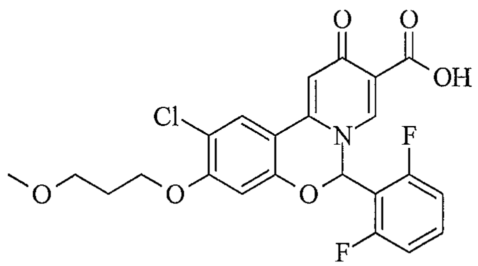

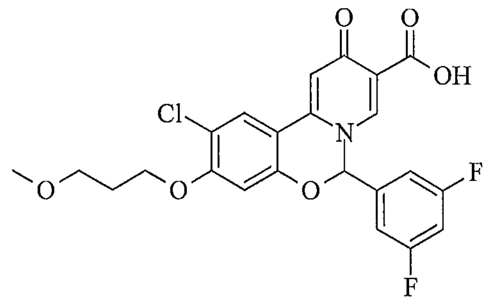

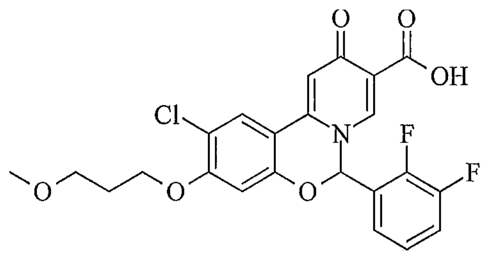

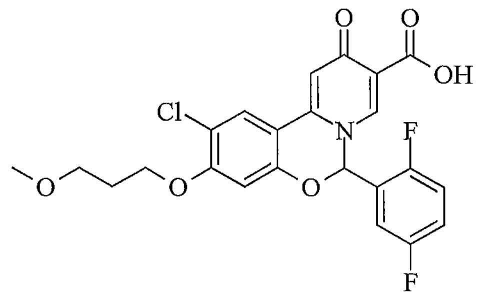

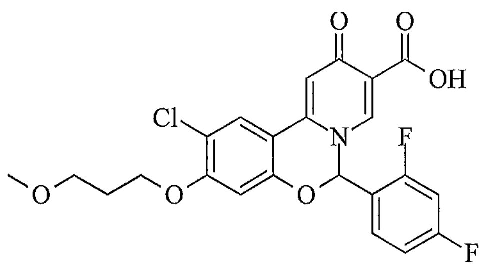

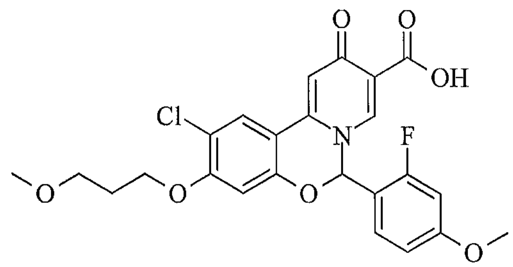

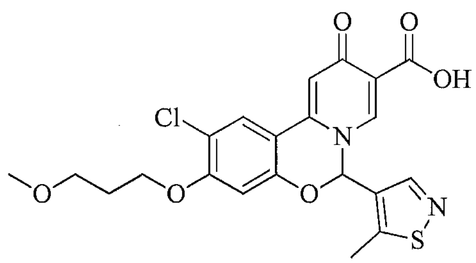

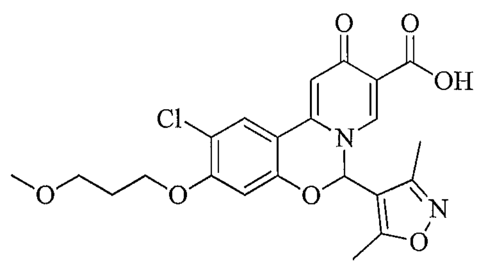

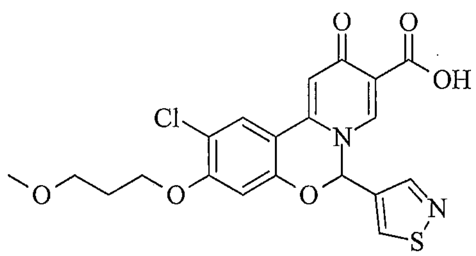

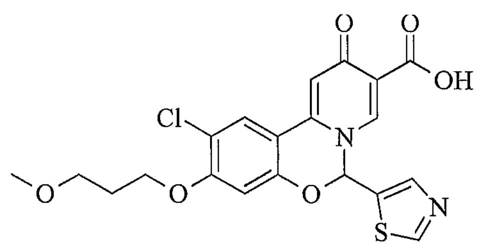

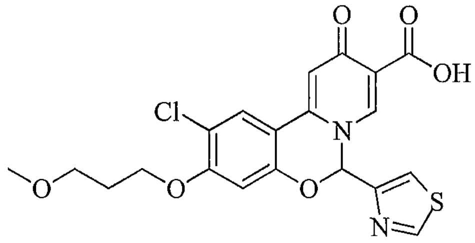

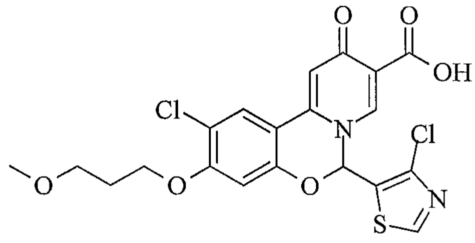

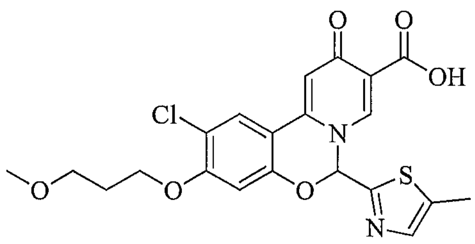

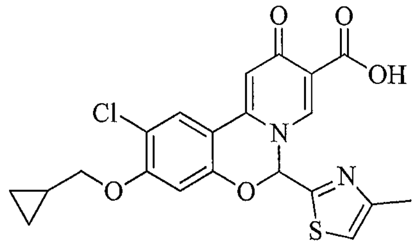

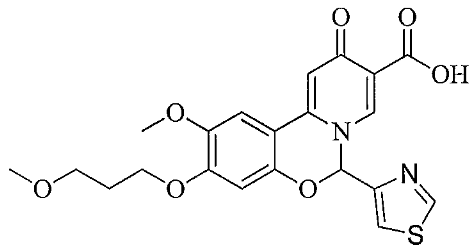

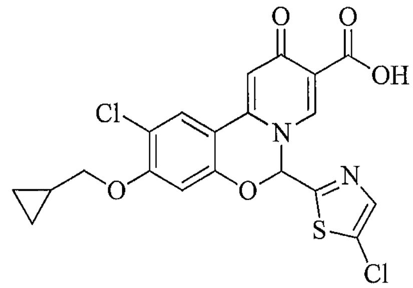

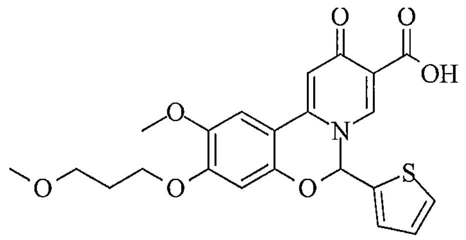

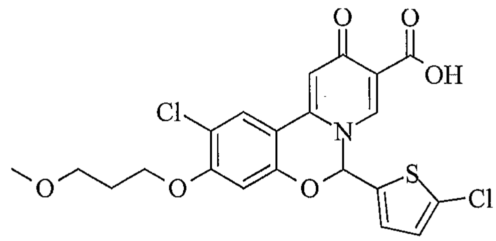

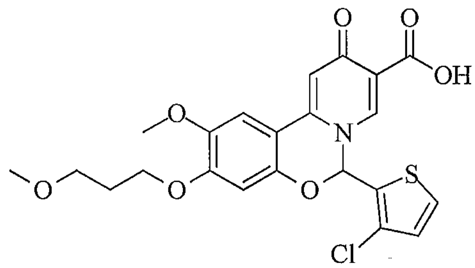

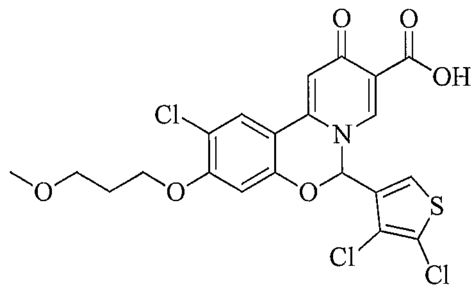

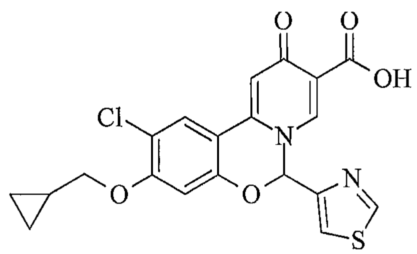

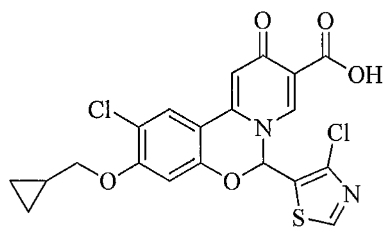

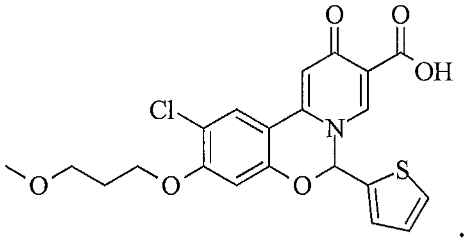

In some embodiments of the present disclosure, in the combination, the compound is selected from:

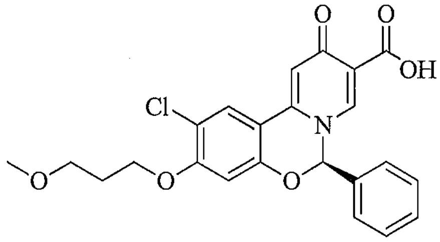

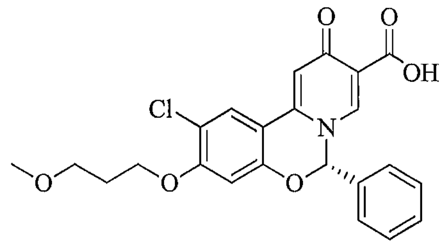

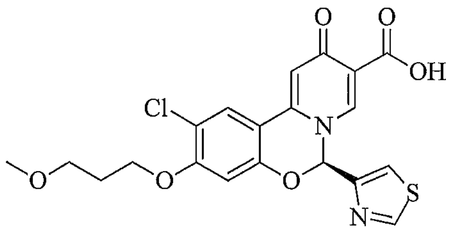

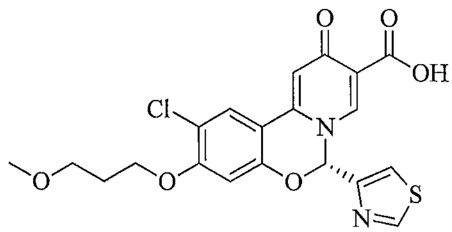

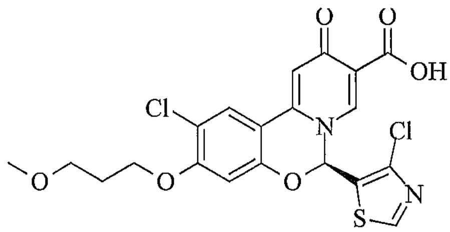

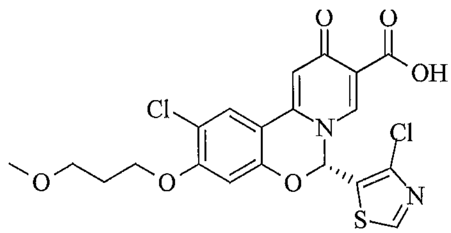

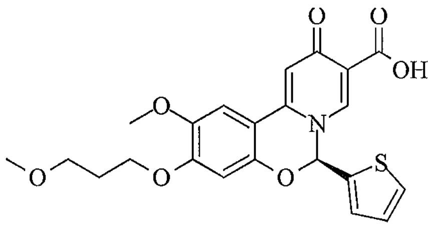

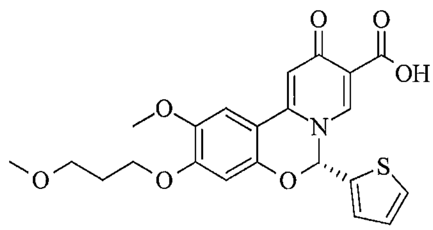

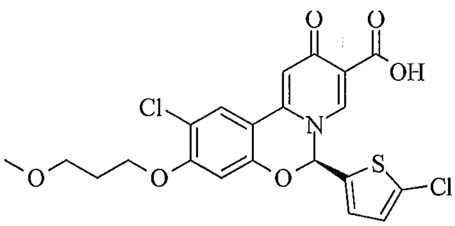

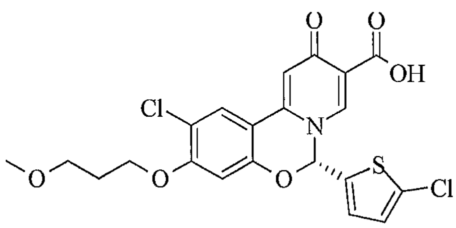

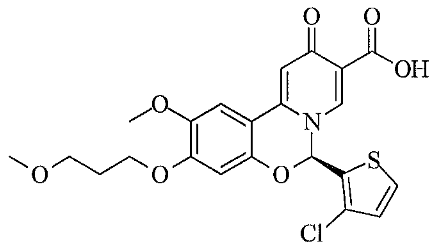

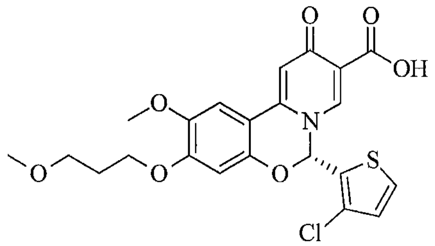

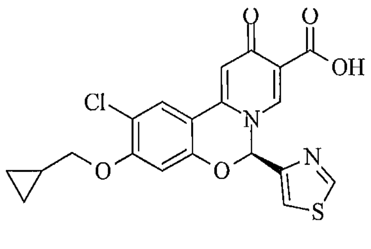

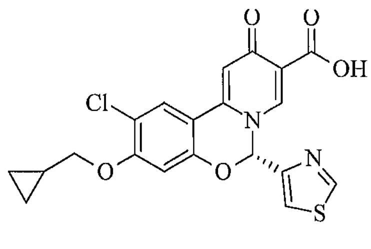

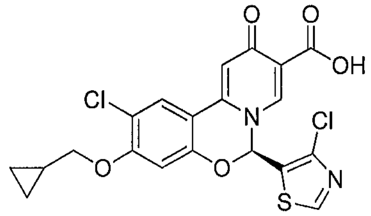

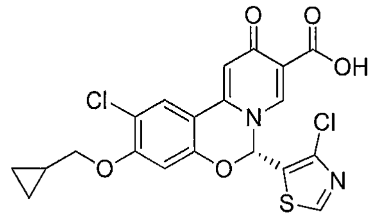

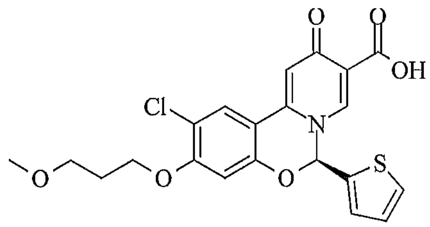

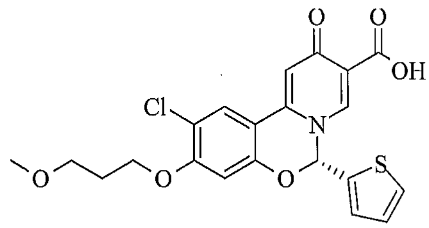

.

The present disclosure also provides use of the combination in the manufacture of a medicament for the treatment of hepatitis B.

The present disclosure also provides a composition, comprising the combination and at least one pharmaceutically acceptable carrier and/or excipient.

The present disclosure also provides a kit, comprising the combination or the composition.

The present disclosure also provides a use of the composition or the kit in the manufacture of a medicament for the treatment of hepatitis B.

In some embodiments of the present disclosure, the combination is a combination for the treatment of chronic hepatitis B.

In some embodiments of the present disclosure, the composition is a pharmaceutical composition for the treatment of chronic hepatitis B.

In some embodiments of the present disclosure, the kit is a kit for the treatment of chronic hepatitis B.

In some embodiments of the present disclosure, the kit also comprises an instruction of the treatment of hepatitis B by a combination of the compound represented by formula (I) or the pharmaceutically acceptable salt thereof with other medicaments for the treatment of hepatitis B.

In some embodiments of the present disclosure, the pharmaceutical composition of the compound represented by formula (I) comprises the compound represented by formula (I) or the pharmaceutically acceptable salt thereof and a pharmaceutically acceptable carrier and/or excipient.

In some embodiments of the present disclosure, the pharmaceutical composition of the compound represented by formula (I) is in the form of a solid preparation, preferably a capsule or a tablet.

In some embodiments of the present disclosure, the pharmaceutical composition of the other medicaments for the treatment of hepatitis B comprises other medicaments for the treatment of hepatitis B and a pharmaceutically acceptable carrier and/or excipient.

In some embodiments of the present disclosure, the pharmaceutical composition of the other medicaments for the treatment of hepatitis B is in the form of a liquid preparation, preferably a water-soluble injection, and the water-soluble injection includes, but is not limited to, a water-soluble preparation not lyophilized or a water-soluble preparation reconstituted from lyophilized powder.

Mode of administration

The following content is not intended to limit the mode of administration of the combination of the present disclosure.
The component of the combination of the present disclosure can be each separately formulated into a pharmaceutical composition, or some or all of them are jointly formulated into a pharmaceutical composition. In some embodiments, the combination of the present disclosure can be formulated into a pharmaceutical composition suitable for single or multiple administration.
The component of the combination of the present disclosure can be administered separately, or some or all of them can be administered jointly. The component of the combination of the present disclosure may not be administered substantially jointly, or some or all of them are administered jointly. The component of the combination of the present disclosure can have the same or different administration cycles.
The components of the combination of the present disclosure can be each administered independently by any suitable variety of routes, including but not limited to, oral or parenteral (by intravenous, intramuscular, subcutaneous, intraperitoneal, spinal or other parenteral administration routes, for example by injection or infusion). In some embodiments, the component of the combination of the present disclosure can each independently be administered orally or by injection, for example by intravenous injection or intraperitoneal injection.
The components of the combination of the present disclosure can each independently be in a suitable dosage form, including but not limited to, tablets, troches, pills, capsules (e.g., hard capsules, soft capsules, enteric capsules, microcapsules), elixirs, granules, syrups, injections (intramuscular, intravenous, intraperitoneal), granules, emulsions, suspensions, solutions, dispersions and sustained-release preparations for oral or non-oral administration.
The components of the combination of the present disclosure can each independently comprise a pharmaceutically acceptable carrier and/or excipient.

## Technical effects

The compound represented by formula (I) of the present disclosure is a hepatitis B surface antigen inhibitor, which can effectively reduce the HBsAg content and has a significant inhibition effect on HBV The combination of the compound represented by formula (I) with the immunomodulator, or the combination of the compound represented by formula (I) with nucleoside or nucleotide reverse transcriptase inhibitors, or the combination of medicaments with these three mechanisms can enhance the effect on HBsAg, play a synergistic inhibitory effect, and simultaneously inhibit HBV virus through multiple channels, so as to achieve the purposes of improving curative effect, reducing toxic and side effects, shortening the treatment period and dosage for administration, reducing drug resistance, etc., and can achieve the effect of reducing the HBV load, reducing or even eliminating HBsAg.

## Definition and description

Unless otherwise indicated, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof. The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.
The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium or similar salts. When the compound of the present disclosure contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for

example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like (refer to Berge et al., "Pharmaceutical Salts", Journal of Pharmaceutical Science 66: 1-19 (1977*)).* Certain specific compounds of the present disclosure contain both basic and acidic functional groups and can be converted to any base or acid addition salt.

Preferably, through bringing the salt into contact with a base or an acid in a conventional manner, then separating the parent compound, the neutral form of the compound is thereby regenerated. The difference between the parent form of the compound and its various salt forms lies in specific physical properties, such as different solubility in a polar solvent.

"Pharmaceutically acceptable salt" used herein belongs to a derivative of the compound of the present disclosure, wherein, the parent compound is modified by forming a salt with an acid or a base. Examples of the pharmaceutically acceptable salt include but are not limited to an inorganic acid or organic acid salt of a basic moiety such as amine, an alkali metal salt or an organic salt of an acidic moiety such as carboxylic acid, and the like. The pharmaceutically acceptable salt includes conventional non-toxic salt or quaternary ammonium salt of the parent compound, such as a salt formed by a non-toxic inorganic acid or an organic acid. The conventional non-toxic salt includes but is not limited to the salt derived from an inorganic acid and an organic acid, wherein the inorganic acid or organic acid is selected from 2-acetoxybenzoic acid, 2-hydroxyethanesulfonic acid, acetic acid, ascorbic acid, benzenesulfonic acid, benzoic acid, bicarbonate, carbonic acid, citric acid, edetic acid, ethanedisulfonic acid, ethanesulfonic acid, fumaric acid, glucoheptose, gluconic acid, glutamic acid, glycolic acid, hydrobromic acid, hydrochloric acid, hydroiodide, hydroxyl, hydroxynaphthalene, isethionic acid, lactic acid, lactose, dodecyl sulfonic acid, maleic acid, malic acid, mandelic acid, methanesulfonic acid, nitric acid, oxalic acid, pamoic acid, pantothenic acid, phenylacetic acid, phosphoric acid, polygalactanal acid, propionic acid, salicylic acid, stearic acid, subacetic acid, succinic acid, sulfamic acid, sulfanilic acid, sulfuric acid, tannin, tartaric acid and *p*-toluenesulfonic acid.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof. Generally, non-aqueous media such as ether, ethyl acetate, ethanol, isopropanol or acetonitrile are preferred.

In addition to the salt form, the compound provided by the present disclosure also exists in prodrug form. The prodrug of the compound described herein is the compound that readily undergoes chemical change under physiological condition to be converted into the compound of the present disclosure. Additionally, the prodrug can be converted to the compound of the present disclosure by a chemical or biochemical method *in vivo* environment.

Certain compounds of the present disclosure can exist in an unsolvated form or a solvated form, including a hydrated form. Generally, the solvated form is equivalent to the unsolvated form, and both are encompassed within the scope of the present disclosure.

Certain compounds of the present disclosure can have an asymmetric carbon atom (optical center) or a double bond. The racemate, diastereomer, geometric isomer and individual isomer are all encompassed within the scope of the present disclosure.

Unless otherwise specified, a wedged bond and a dashed bond (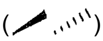) are used to indicate the absolute configuration of a stereogenic center, 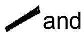 and 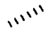 are used to indicate the relative configuration of a stereogenic center. When the compound described herein contains an olefinic double bond or other geometric asymmetric centers, *E* and *Z* geometric isomers are included unless otherwise specified. Likewise, all tautomeric forms are encompassed within the scope of the present disclosure.

The compound of the present disclosure may present in a specific geometric or stereoisomeric form. The present disclosure contemplates all such compounds, including *cis* and *trans* isomer, (-)- and (+)-enantiomer, (*R*)- and (*S*)-enantiomer, diastereoisomer, (*D*)-isomer, (*L*)-isomer, and racemic mixture and other mixtures, for example, an enantiomer or diastereoisomer enriched mixture, all of which are encompassed within the scope of the present disclosure. The substituent such as alkyl may have an additional asymmetric carbon atom. All these isomers and mixtures thereof are encompassed within the scope of the present disclosure.

Optically active (*R*)- and (*S*)-isomer, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound

reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (for example, carbamate generated from amine).

The compound of the present disclosure may contain an unnatural proportion of atomic isotope at one or more than one atom (s) that constitute the compound. For example, the compound can be radiolabeled with a radioactive isotope, such as tritium ($^3H$), iodine-125 ($^{125}I$) or C-14 ($^{14}C$). All isotopic variations of the compound of the present disclosure, whether radioactive or not, are encompassed within the scope of the present disclosure.

The term "pharmaceutically acceptable carrier" refers to any preparations or carrier medium capable of delivering an effective amount of the active substance of the present disclosure, without interfering with the biological activity of the active substance, and without toxic side effects to the host or patient, including water, oil, vegetable and mineral, cream base, lotion base, ointment base, etc. These bases include suspension, tackifier, transdermal accelerator, etc. Their preparations are well known to those skilled in the field of cosmetics or topical medicine. For other information about the carrier, please refer to Remington: The Science and Practice of Pharmacy, 21st Ed., Lippincott, Williams & Wilkins (2005), the contents of which are incorporated herein by reference.

For a medicament or pharmacologically active agent, the term "effective amount" or "therapeutically effective amount" refer to an adequate dosage of a medicament or agent that is non-toxic but achieves the desired effect. For oral dosage forms of the present disclosure, the "effective amount" of one active substance in the composition refers to the amount required to achieve the desired effect when combined with another active substance in the composition. The determination of the effective amount varies from person to person, depending on the age and general condition of the recipient, but also depends on the specific active substance, and the appropriate effective amount in each case may be determined by those skilled in the art on the basis of routine tests.

The terms "active ingredient", "therapeutic agent", "active substance" or "active agent" refer to a chemical entity that is effective in the treatment of a target disorder, disease or disorder.

The term "optional" or "optionally" means that the subsequent event or condition may occur but not requisite, that the term includes the instance in which the event or condition occurs and the instance in which the event or condition does not occur.

The term "substituted" means one or more than one hydrogen atom (s) on a specific atom are substituted by a substituent, including deuterium and hydrogen variants, as long as the valence of the specific atom is normal and the substituted compound is stable. When the substituent is a keto group (i.e., =O), it means two hydrogen atoms are substituted. Positions on an aromatic ring cannot be substituted by a keto group. The term "optionally substituted" means an atom can be substituted by a substituent or not, unless otherwise specified, the species and number of the substituent may be arbitrary as long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variables is a single bond, it means that the two groups linked by the single bond are connected directly. For example, when L in A-L-Z represents a single bond, the structure of A-L-Z is actually A-Z.

When a substituent is vacant, it means that the substituent does not exist. For example, when X is vacant in A-X, the structure of A-X is actually A. When a bond of a substituent can be cross-linked to two atoms on a ring, such substituent can be bonded to any atom on the ring. When an enumerative substituent does not indicate by which atom it is attached to a compound included in the general chemical formula but not specifically mentioned, such substituent can be bonded by any of its atoms. A combination of substituents and/or variants thereof is allowed only when such combination can result in a stable compound. For example, the structural unit

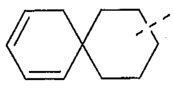
or
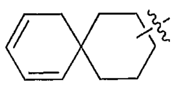

means that it can be substituted at any position on cyclohexyl or cyclohexadiene.

Unless otherwise specified, the term "hetero" represents a heteroatom or a heteroatom group (e.g., an atom group containing a heteroatom), including the atom except carbon (C) and hydrogen (H) and the atom group containing the above heteroatom, for example, including oxygen (O), nitrogen (N), sulfur (S), silicon (Si), germanium (Ge), aluminum (Al), boron (B), -O-, -S-, =O, =S, -C(=O)O-, -C(=O)-, -C(=S)-, -S(=O), $-S(=O)_2-$, and the group consisting of - C(=O)N(H)-, -N(H)-, -C(=NH)-, $-S(=O)_2N(H)-$ and -S(=O)N(H)-, each of which is optionally substituted.

Unless otherwise specified, the term "ring" refers to a substituted or unsubstituted cycloalkyl, heterocycloalkyl, cycloalkenyl, heterocycloalkenyl, cycloalkynyl, heterocycloalkynyl, aryl or heteroaryl. The so-called ring includes a single ring, a ring assembly, a spiral ring, a fused ring or a bridged ring. The number of the atom on the ring is usually defined as the member number of the ring, for example, a "5-7 membered ring" means that 5 to 7 atoms are arranged on a ring. Unless otherwise specified, the ring optionally contains 1 to 3 heteroatoms. Therefore, a "5-7 membered ring" includes, for example, phenyl, pyridinyl and piperidinyl; on the other hand, the term "5-7 membered heterocycloalkyl ring" includes pyridyl and piperidinyl, but excluding phenyl. The term "ring" also includes a ring system containing at least one ring, wherein each ring independently meets the above definition.

Unless otherwise specified, the term "heterocycle" or "heterocyclyl" refers to a stable monocyclic, bicyclic or tricyclic ring containing a heteroatom or a heteroatom group, which can be saturated, partially unsaturated or unsaturated (aromatic) and can contain carbon atoms and 1, 2, 3 or 4 ring heteroatoms independently selected from N, O and S, wherein any of the above heterocycle can be fused to a benzene ring to form a bicyclic ring. Nitrogen and sulfur heteroatoms can optionally be oxidized (i.e., NO and S $(O)_p$, p is 1 or 2). Nitrogen atom can be substituted or unsubstituted (i.e., N or NR, wherein R is H or other substituents already defined herein). The heterocycle can be attached to the pendant group of any heteroatom or carbon atom to form a stable structure. If the resulting compound is stable, the heterocycle described herein may have a displacement at a carbon or nitrogen position. Nitrogen atom on the heterocycle is optionally quaternized. In a preferred embodiment, when the total number of S and O atom of the heterocycle is more than 1, the heteroatom is not adjacent to each other. In another preferred embodiment, the total number of S and O atom of the heterocycle is not more than 1.

As used herein, the term "aromatic heterocyclic group" or "heteroaryl" refers to a stable 5-, 6- or 7-membered monocyclic or bicyclic or 7-, 8-, 9- or 10-membered bicyclic heterocyclic aromatic ring which contains carbon atoms and 1, 2, 3 or 4 ring heteroatoms independently selected from N, O and S. Nitrogen atom can be substituted or unsubstituted (i.e., N or NR, wherein R is H or other substituents already defined herein). Nitrogen and sulfur heteroatoms may optionally be oxidized (i.e., NO and S $(O)_p$, p is 1 or 2). It is worth noting that the total number of S and O atom of an aromatic heterocycle is not more than one. The bridged ring is also included in the definition of the heterocycle. A bridged ring is formed when one or more than one atom (i.e., C, O, N or S) link two non-adjacent carbon or nitrogen atoms. A preferred bridged ring includes, but not limited to one carbon atom, two carbon atoms, one nitrogen atom, two nitrogen atoms and one carbon-nitrogen group. It is worth noting that a bridge always converts a monocyclic ring to a tricyclic ring. In a bridged ring, the substituent on the ring may also be present on the bridge.

Examples of the heterocyclic compound include, but are not limited to: acridinyl, azocinyl, benzimidazolyl, benzofuranyl, benzomercaptofuranyl, benzomercaptophenyl, benzoxazolyl, benzoxazolinyl, benzothiazolyl, benzotriazolyl, benzotetrazolyl, benzoisoxazolyl, benzoisothiazolyl, benzoimidazolinyl, carbazolyl, 4a*H*-carbazolyl, carbolinyl, chromanyl, chromene, cinnolinyl decahydroquinolinyl, 2*H*,6*H*-1,5,2-dithiazinyl, dihydrofuro[2,3-*b*]tetrahydrofuranyl, furanyl, furazanyl, imidazolidinyl, imidazolinyl, imidazolyl, 1*H*-indazolyl, indolenyl, indolinyl, indolizinyl, indolyl, 3*H*-indolyl, isobenzofuranyl, isoindolyl, isoindolinyl, isoquinolinyl, isothiazolyl, isoxazolyl, methylenedioxyphenyl, morpholinyl, naphthyridinyl, octahydro-isoquinolinyl, oxadiazolyl, 1, 2,3-oxadiazolyl, 1,2,4-oxadiazolyl, 1,2,5-oxadiazolyl, 1,3,4-oxadiazolyl, oxazolidinyl, oxazolyl, hydroxindolyl, pyrimidinyl, phenanthridinyl, phenanthrolinyl, phenazine, phenothiazine, benzoxanthinyl, phenoloxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyrido-oxazolyl, pyrido-imidazolyl, pyrido-thiazolyl, pyridinyl, pyrrolidinyl, pyrrolinyl, 2*H*-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, tetrazolyl, 6*H* 1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, isothiazolylthienyl, thienyl, thieno-oxazolyl, thieno-thiazolyl, thieno-imidazolyl, thienyl, triazinyl, 1,2,3-triazolyl, 1,2,4-triazolyl, 1,2,5-triazolyl, 1,3,4-triazolyl and xanthenyl. Fused-ring compounds and spiro compounds are also included.

Unless otherwise specified, the term "hydrocarbyl" or its hyponyms (e.g., alkyl, alkenyl, alkynyl, and aryl), by itself or as part of another substituent, refers to a linear, branched chain or cyclic hydrocarbon radical or any combination thereof. They can be fully saturated (e.g., alkyl), mono- or polyunsaturated (e.g., alkenyl, alkynyl, and aryl), can be mono-, or poly-substituted, can be monovalent (e.g., methyl), divalent (e.g., methylene) or multivalent (e.g., methenyl), can also include a divalent or multivalent group, have a specified number of carbon atom (e.g., $C_1$-$C_{12}$ indicates 1 to 12 carbon atoms, $C_{1-12}$ is selected from $C_1$, $C_2$, $C_3$, $C_4$, $C_5$, $C_6$, $C_7$, $C_8$, $C_9$, $C_{10}$, $C_{11}$ and $C_{12}$; $C_{3-12}$ is selected from $C_3$, $C_4$, $C_5$, $C_6$, $C_7$, $C_8$, $C_9$, $C_{10}$, $C_{11}$ and $C_{12}$). The term "hydrocarbyl" includes, but is not limited to aliphatic hydrocarbyl and aromatic hydrocarbyl. The aliphatic hydrocarbyl includes linear and cyclic hydrocarbyl, specifically includes but not limited to alkyl, alkenyl, and alkynyl. The aromatic hydrocarbyl includes but is not limited to 6- to 12-membered aromatic hydrocarbyl such as phenyl, naphthyl and the like. In some embodiments, the term "hydrocarbyl" refers to a linear or branched group or a combination thereof which can be fully saturated, mono- or polyunsaturated, and can include a divalent or multivalent group. Examples of the saturated hydrocarbyl group include, but are not limited to, methyl, ethyl, *n*-propyl, isopropyl, *n*-butyl, *tert*-butyl, isobutyl, *sec*-butyl, cyclohexyl, (cyclohexyl)methyl, cyclopropylmethyl, and the homolog or isomer of *n*-amyl, *n*-hexyl, *n*-heptyl, *n*-octyl and other atom groups. The unsaturated hydrocarbyl has one

or more than one double or triple bonds. Examples of the unsaturated alkyl include but are not limited to, vinyl, 2-propenyl, butenyl, crotyl, 2-isopentenyl, 2-(butadienyl), 2,4-pentadienyl, 3-(1,4-pentadienyl), ethynyl, 1- and 3-propynyl, 3-butynyl, and more higher homologs and isomers.

Unless otherwise specified, the term "heterohydrocarbyl" or its hyponyms (e.g., heteroalkyl, heteroalkenyl, heteroalkynyl, and heteroaryl), by itself or as part of another substituent, refers to a stable linear, branched or cyclic hydrocarbon group or any combination thereof, which has a specified number of carbon atoms and at least one heteroatom. In some embodiments, the term "heteroalkyl" by itself or in combination with another term refers to a stable linear chain, branched hydrocarbon radical or a combination thereof which has a specified number of carbon atoms and at least one heteroatom. In a specific embodiment, a heteroatom is selected from B, O, N and S, wherein nitrogen and sulfur atoms are optionally oxidized and the nitrogen atom is optionally quaternized. The heteroatom or heteroatom group can be located at any interior position of a heterohydrocarbyl, including the position where the hydrocarbyl attaches to the rest part of the molecule. But the terms "alkoxy", "alkylamino" and "alkylthio" (or thioalkyl) are used by the conventional meaning and refer to an alkyl connected to the rest part of the molecule via an oxygen atom, an amino or a sulfur atom respectively. Examples include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2$, $-S(O)-CH_3$, $-CH_2-CH_2-S(O)_2-CH_3$, $-CH=CH-O-CH_3$, $-CH_2-CH=N-OCH_3$ and $-CH=CH-N(CH_3)-CH_3$. Up to two consecutive heteroatoms can be present, such as, $-CH_2-NH-OCH_3$.

Unless otherwise specified, the term "cyclohydrocarbyl", "heterocyclohydrocarbyl" or its hyponyms (e.g., aryl, heteroaryl, cycloalkyl, heterocycloalkyl, cycloalkenyl, heterocycloalkenyl, cycloalkynyl, heterocycloalkynyl) by itself or in combination with another term refers to cyclized "hydrocarbyl" or "heterohydrocarbyl". Furthermore, for heterohydrocarbyl or heterocyclohydrocarbyl (e.g., heteroalkyl, and heterocycloalkyl), one heteroatom can occupy the position where the heterocycle attaches to the remainder position of the molecule. Examples of the cycloalkyl include, but are not limited to, cyclopentyl, cyclohexyl, 1-cyclohexenyl, 3-cyclohexenyl, cycloheptyl and the like. Non-limiting examples of heterocycloalkyl include 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-indol-3-yl, tetrahydro-thiophen-2-yl, tetrahydro-thiophen-3-yl, 1-piperazinyl and 2-piperazinyl.

Unless otherwise specified, the term "alkyl" refers to a linear chain or branched saturated hydrocarbon group, can be mono-substituted (e.g., $-CH_2F$) or poly-substituted (e.g., $-CF_3$), can be monovalent (e.g., methyl), divalent (e.g., methylene) or multivalent (e.g., methenyl). Examples of alkyl include methyl (Me), ethyl (Et), propyl (e.g., *n*-propyl and isopropyl), butyl (e.g., *n*-butyl, isobutyl, *s*-butyl, *t*-butyl), pentyl (e.g., *n*-pentyl, isopentyl, neopentyl) and the like.

Unless otherwise specified, the term "alkynyl" refers to an alkyl group having one or more than one carbon-carbon triple bonds at any position on the chain, can be mono-substituted or poly-substituted, and can be monovalent, divalent or multivalent. Examples of alkynyl include ethynyl, propynyl, butynyl, pentynyl, and the like.

Unless otherwise specified, cycloalkyl includes any stable cyclic or polycyclic hydrocarbyl, and any carbon atom is saturated, can be mono-substituted or poly-substituted, and can be monovalent, divalent or multivalent. Examples of cycloalkyl include, but are not limited to, cyclopropyl, norbornanyl, [2.2.2]bicyclooctane, [4.4.0]bicyclodecanyl and the like.

Unless otherwise specified, the term "halo" or "halogen" by itself or as part of another substituent refers to fluorine, chlorine, bromine or iodine atom. Furthermore, the term "haloalkyl" is meant to include monohaloalkyl and polyhaloalkyl. For example, the term "halo ($C_1$-$C_4$)alkyl" is meant to include, but not limited to, trifluoromethyl, 2,2,2-trifluoroethyl, 4-chlorobutyl, 3-bromopropyl and the like. Unless otherwise specified, examples of haloalkyl include, but not limited to trifluoromethyl, trichloromethyl, pentafluoroethyl and pentachloroethyl.

The term "alkoxy" represents any alkyl defined above having a specified number of carbon atoms attached by an oxygen bridge. Unless otherwise specified, $C_{1-6}$ alkoxy includes $C_1$, $C_2$, $C_3$, $C_4$, $C_5$ and $C_6$ alkoxy. Examples of alkoxy include, but not limited to methoxy, ethoxy, *n*-propoxy, isopropoxy, *n*-butoxy, *sec*-butoxy, *tert*-butoxy, *n*-pentyloxy and *S*-pentoxy.

Unless otherwise specified, the term "aryl" refers to a polyunsaturated aromatic substituent, can be mono- or poly-substituted, can be a monovalent, divalent or multivalent, can be a single ring or a multiple ring (e.g., one to three rings; wherein at least one ring is aromatic), which are fused together or connected covalently. The term "heteroaryl" refers to an aryl (or ring) containing one to four heteroatoms. In an illustrative example, the heteroatom is selected from B, O, N and S, wherein nitrogen and sulfur atoms are optionally oxidized and nitrogen atom is optionally quaternized. A heteroaryl may attach to the rest part of a molecule via a heteroatom. Non-limiting examples of aryl or heteroaryl include phenyl, naphthyl, biphenyl, pyrrolyl, pyrazolyl, imidazolyl, pyrazinyl, oxazolyl, phenyl-oxazolyl, isoxazolyl, thiazolyl, furyl, thienyl, pyridyl, pyrimidinyl, benzothiazolyl, purinyl, benzimidazolyl, indolyl, isoquinolinyl, quinoxalinyl, quinolyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl and 6-quinolyl. The substituent of any of the above aryl and heteroaryl ring system is selected from the acceptable substituent described

below.

Unless otherwise specified, when combined with other terms (such as aryloxy, arylthio, arylalkyl), the aryl includes the aryl and heteroaryl ring as defined above. Thus, the term "aralkyl" is meant to include the group (e.g., benzyl, phenethyl, pyridylmethyl) where an aryl is attached to an alkyl, including an alkyl where the carbon atom (e.g., methylene) has been replaced by an atom such as oxygen, for example, phenoxymethyl, 2-pyridyloxy, 3-(1-naphthyloxy)propyl, and the like.

DETAILED DESCRIPTION OF THE PREFERRED EMBODIMENT

The present disclosure is described in detail by the embodiments below, but it does not mean that there are any adverse restrictions on the present disclosure. The present disclosure has been described in detail herein, wherein specific embodiments thereof are also disclosed, and it will be apparent to those skilled in the art that various variations and improvements can be made to specific embodiments of the present disclosure without departing from the spirit and scope of the present disclosure.

**Embodiment 1**

Experimental objective: The purpose of this study was to evaluate the *in vitro* anti-hepatitis B virus efficacy of the test compounds in combination with TAF (tenofovir alafenamide fumarate) and Peg-IFN-α2a (peginterferon α 2a) using human primary hepatocytes (PHH) isolated from a humanized FRG mouse model.

Experimental materials:

(1) Main instrument: fluorescence qPCR instrument (Applied Biosystems, model 7500). Microplate reader (Bio Tek, model Synergy 2). Chemiluminescence imaging system (GE, model LAS4010)

(2) Main reagents and consumables: FastStart Universal Probe Master (Roche), DNA extraction kit (Qiagen), 96-well plate (Costar 3599) and 96-well V-type plate (Axygen WIPP02280), HBsAg ELISA kit (Autobio) and HBeAg ELISA kit (Autobio), Cell Counting Kit-8 (CCK-8) (Biolite).

(3) Others: FRG mice, D-type HBV, test compound WWS0

( 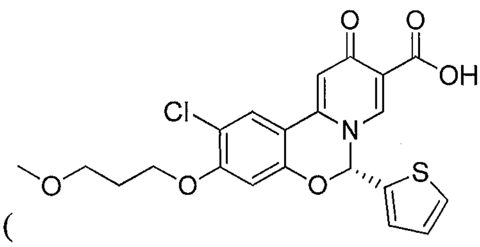 ).

Experimental method:

1. The PHH model was used to evaluate the single drug *in vitro* antiviral activity of the test compound

On day 0, primary human hepatocytes were resuscitated and plated into two 48-well plates ($1.32\times10^5$ cells/well).

On day 1, HBV was added to infect PHH (2000 GE/cell). Two kinds of compound treatment methods were set up: One cell plate was treated with the test compound and Peg-IFNα-2a for four hours on day 1, and then the medium was then replaced with an HBV-containing medium to infect cells (at the same time, compounds were added to continue treatment); another plate was treated with test compound and Peg-IFNα-2a started on day 2. Fresh medium with compound was replaced every 1 to 2 days.

The final concentration of DMSO in the medium was 2%. The initial concentration of the test compound was 1000 nM, and the initial concentration of the control compound Peg-IFNα-2a was 500 IU/mL with 5-fold dilution, 7 concentration points, and three duplicate wells (see Table 3 for compound concentration settings).

The medium was replaced with the fresh medium containing the compound on days 4, 6, 8, 10, 12, 14, 16, 18 and 20. On day 22, the culture supernatant was collected and cell viability was detected using CellTiter-Glo.

Cell culture supernatants on days 8, 14 and 22 were collected for HBV DNA, HBeAg and HBsAg detection.

**Table 1 Individual administered concentration settings of the test compound**

| Compound | | Concentration | | | | | | | | Solvent |
|---|---|---|---|---|---|---|---|---|---|---|
| | | 7 | 6 | 5 | 4 | 3 | 2 | 1 | Unit | |
| a | Test compound | 1000 | 200 | 40 | 8 | 1.6 | 0.32 | 0.064 | nM | DMSO |
| b | Peg-IFNα-2a | 500 | 100 | 20 | 4 | 0.8 | 0.16 | 0.032 | IU/mL | Medium |

2. The PHH model was used to evaluate the *in vitro* anti-HBV activity of the test compound in combination with TAF and Peg-IFNα-2a

On day 0, primary human hepatocytes were resuscitated and plated into six 48-well plates ($1.32\times10^5$ cells/well).

On day 1, PHH was added to infect HBV (2000 GE/cell). On the second day, different groups of combined drugs were added for treatment:

(1) Test compound administered in combination with Peg-IFNα-2a: Both the test compound and Peg-IFN-α2a were diluted 3-fold at 5 concentration points, tested in a 5×5 matrix, and three duplicate plates. The starting concentrations of the test compound and Peg-IFNα-2a were 400 nM and 100 IU/mL, respectively. The arrangement of the compounds in the combined action is shown in Table 2, and the test concentrations of the compounds are shown in Table 3.

The final concentration of DMSO in the cell medium was 2%. The medium was replaced with the medium containing the compound on days 4 and 6. On day 8, the culture supernatant was collected for detecting HBV DNA, HBeAg and HBsAg, and then the collected supernatant was added with CellTiter-Glo to measure cell viability.

**Table 2 Administered arrangement of the test compound in combination with Peg-IFNα-2a**

| | 1 | 2 | 3 | 4 | 5 | 6 | 7 | 8 |
|---|---|---|---|---|---|---|---|---|
| A | a0;b5 | a5;b5 | a4;b5 | a3;b5 | a2;b5 | a1;b5 | DMSO | Uninf |
| B | a0;b4 | a5;b4 | a4;b4 | a3;b4 | a2;b4 | a1;b4 | DMSO | Uninf |
| C | a0;b3 | a5;b3 | a4;b3 | a3;b3 | a2;b3 | a1;b3 | DMSO | Uninf |
| D | a0;b2 | a5;b2 | a4;b2 | a3;b2 | a2;b2 | a1;b2 | DMSO | Uninf |
| E | a0;b1 | a5;b1 | a4;b1 | a3;b1 | a2;b1 | a1;b1 | DMSO | Uninf |
| F | DMSO | a5;b0 | a4;b0 | a3;b0 | a2;b0 | a1;b0 | DMSO | Uninf |
| a=test compound, b=Peg-IFNα-2a; 0 means no compound, 1-5 means 5 concentrations | | | | | | | | |

**Table 3 Administered concentration settings of the test compound in combination with Peg-IFNα-2a**

| **Compound** | | **Concentration** | | | | | | **Solvent** |
|---|---|---|---|---|---|---|---|---|
| | | 5 | 4 | 3 | 2 | 1 | Unit | |
| a | Test compound | 400 | 133.33 | 44.44 | 14.81 | 4.94 | nM | DMSO |
| b | Peg-IFNα-2a | 100 | 33.33 | 11.11 | 3.70 | 1.23 | IU/mL | Medium |

(2) Test compound administered in combination with TAF and Peg-IFNα-2a: Both the test compound and Peg-IFNα-2a were diluted 3-fold at 5 concentration points, tested in a 5×5 matrix, and three duplicate plates. The starting concentrations of the test compound and Peg-IFNα-2a were 400 nM and 100 IU/mL, respectively, and TAF was set a concentration point of 0.3 nM. The arrangement of the compounds in the combined action is shown in Table 4, and the test concentrations of the compounds are shown in Table 5.

The final concentration of DMSO in the medium was 2%. The medium was replaced with the medium containing the compound on days 4 and 6. On day 8, the culture supernatant was collected for detecting HBV DNA, HBeAg and HBsAg, and then the collected cell supernatant was added with CellTiter-Glo to measure cell viability.

**Table 4 Administered arrangement of the test compound in combination with TAF and Peg-IFNα-2a**

| | 1 | 2 | 3 | 4 | 5 | 6 | 7 | 8 |
|---|---|---|---|---|---|---|---|---|
| A | a0;b5;c | a5;b5;c | a4;b5;c | a3;b5;c | a2;b5;c | a1;b5;c | DMSO | Uninf |
| B | a0;b4;c | a5;b4;c | a4;b4;c | a3;b4;c | a2;b4;c | a1;b4;c | DMSO | Uninf |
| C | a0;b3;c | a5;b3;c | a4;b3;c | a3;b3;c | a2;b3;c | a1;b3;c | DMSO | Uninf |
| D | a0;b2;c | a5;b2;c | a4;b2;c | a3;b2;c | a2;b2;c | a1;b2;c | DMSO | Uninf |
| E | a0;b1;c | a5;b1;c | a4;b1;c | a3;b1;c | a2;b1;c | a1;b1;c | DMSO | Uninf |
| F | DMSO | a5;b0;c | a4;b0;c | a3;b0;c | a2;b0;c | a1;b0;c | DMSO | Uninf |
| a=test compound, b=Peg-IFNα-2a, c=TAF; 0 means no compound, 1 to 5 means 5 concentrations, Uninf means uninfected control group | | | | | | | | |

**Table 5 Administered concentration settings of the test compound in combination with TAF and Peg-IFNα-2a**

| Compound | | Concentration | | | | | | Solvent |
|---|---|---|---|---|---|---|---|---|
| | | 5 | 4 | 3 | 2 | 1 | Unit | |
| a | The subject compound | 400 | 133.33 | 44.44 | 14.81 | 4.94 | nM | DMSO |
| b | Peg-IFNα-2a | 100 | 33.33 | 11.11 | 3.70 | 1.23 | IU/mL | Medium |
| c | TAF | 0.3 | | | | | nM | DMSO |

3. Sample detection

Cell viability detection: After collecting the cell culture supernatant, CellTiter-Glo was diluted with medium at a ratio of 1:1, and added to the cell plate, and left at room temperature for 10 minutes to detect the luminescence value with a microplate reader.

HBV DNA detection: DNA in the supernatant was extracted according to the DNA extraction kit (Qiagen-51162), and HBV DNA in the sample was quantified by qPCR method, and the qPCR reaction system is shown in Table 6. HBV plasmid DNA was used as a standard for 10-fold serial dilution, and the standard range was from $1.0\times10^7$ copies/μL to 10 copies/μL. The PCR reaction program was: Heating at 95°C for 10 minutes, then cycle mode was entered, and heating at 95°C for 15 seconds, then at 60°C for 1 minute for a total of 40 cycles. The HBV DNA content in the samples was calculated based on the standard curve and the Ct value of each sample.

**Table 6 qPCR reaction system**

| Reagent | Volume required to configure 1 well (μL) |
|---|---|
| Quantitative and rapid initiation of universal probe reagents | 5 |
| Forward primer (10 μM) | 0.4 |
| Reverse primer (10 μM) | 0.4 |
| Probe (10 μM) | 0.2 |
| AE solvent | 2 |
| DNA in Sample | 2 |

Detection of HBeAg and HBsAg: According to the instructions of ELISA kit, the levels of HBeAg and HBsAg in supernatant were detected. The method was briefly described as follows: 50 μL of standard was taken, and sample and control sample were added into the detection plate, then 50 μL of enzyme conjugate was added to each well, incubated at 37°C for 60 minutes, and washed with a washing solution and then dried, and then 50 μL of pre-mixed luminescent

substrate was added and the mixture was incubated at room temperature for 10 minutes in the dark, and finally the luminescence value was measured by a microplate reader. The content of HBeAg and HBsAg in the sample was calculated according to the standard curve and the chemiluminescence value of each sample.

4. Data processing and statistical analysis

$$\text{Cell activity\%} = (\text{luminescence value of sample-average value of blank control well})/(\text{luminescence value of DMSO control group-average value of blank control well}) \times 100$$

$$\text{Inhibition rate \%}=(1- \text{HBV DNA content or HBsAg content or HBeAg content in the sample/HBV DNA content or HBsAg content or HBeAg content in DMSO control group}) \times 100$$

$CC_{50}$ and $EC_{50}$ were calculated by GraphPad Prism software, and the inhibition curve fitting method was sigmoidal dose-response (variable slope).

Combined efficacy was analyzed by MacSynergy™ II software. The combined efficacy was calculated according to a confidence interval of 95% according to the synergism/antagonism, and the results were explained as follows according to MacSynergy criteria:

<25 = Insignificant synergism/antagonism (additive);
25-50 = Minor but significant synergism/antagonism;
50-100 = Moderate synergism/antagonism - may be important *in vivo*;
>100 = Strong synergism/antagonism - probably important *in vivo*;
about 1000 = Possible errors - check data and repeat experiment;
5. Experimental results: See Tables 7 and 8.

**Table 7 Anti-HBV activity and cytotoxicity of single drug in PHH (48-well culture plate)**

| Compound | Administration after infection | | | | Administration before infection | | | | Unit |
|---|---|---|---|---|---|---|---|---|---|
| | $EC_{50}$ (Day 8) | | | $CC_{50}$ | $EC_{50}$ (Day 8) | | | $CC_{50}$ | |
| | HBV DNA | HBeAg | HBsAg | Day 22 | HBV DNA | HBeAg | HBsAg | Day 22 | |
| WWS0 | 45.52 | 32.35 | 47.34 | >1000 | 13.47 | 25.99 | 47.15 | >1000 | nM |
| Peg-IFNα-2a | 0.81 | 13.5 | 42.76 | >500 | 3.59 | 8.53 | 23.49 | 77.56 | IU/mL |

**Table 8 Results of combined administration of the test compound in PHH (95% confidence interval)**

| Combination | HBsAg | | | HBeAg | | |
|---|---|---|---|---|---|---|
| | Synergistic index | Antagonistic index | Result | Synergistic index | Antagonistic index | Result |
| WWS0+ Peg-IFNα-2a | 37.76 | 0 | Moder ate coordi nation | 22.43 | 0 | additio n |
| WWS0 + Peg-IFNα-2a + TAF | 36.47 | 0 | Moder ate coordi nation | 0.78 | -0.8 | additio n |

6. Experimental conclusion: In the PHH *in vitro* infection model of HBV infection, the test compound WWS0 inhibits HBV DNA, HBeAg and HBsAg in a dose-dependent manner, and the replication activity of the inhibition of HBV by WWS0 is basically the same in the two modes of administration before and after HBV infection.

The combined administration result of the test compound shows that the combined administration of WWS0 and Peg-IFNα-2a has a moderate synergism on the inhibition of HBsAg, and additive effect on the inhibition of HBeAg. The three-drug combination of test compound WWS0 with TAF and Peg-IFNα-2a, has a moderate synergism on the inhibition of HBsAg and additive effect on the inhibition of HBeAg.

In the test compound WWS0 single drug and combined action experiment, no obvious cytotoxicity is displayed within the test concentration range.

## Embodiment 2

In this study, HepG2-NTCP cell *in vitro* infection model was used to evaluate the inhibitory activity of a combined administration of compound WWS0 with Entecavir (ETV) on HBV. The content of HBV DNA in cell supernatant was detected by real-time quantitative PCR (qPCR) method, and the content of HBeAg and HBsAg was detected by ELISA, and the anti-HBV activity of the combined action of the compound was determined, while CellTiter-Glo was used to detect the effect of the test compound on cell viability.

### 1. Experimental materials:

Cell line: HepG2-NTCP cell line was constructed and provided by WuXi AppTec. HepG2-NTCP cells were HepG2 cells stably transfected and continued to highly express the human NTCP gene, and were susceptible to be infected by HBV. The cell medium was DMEM medium added with 10% fetal bovine serum, 1% Penicillin-Streptomycin, 1% glutamine, 1% non-essential amino acids and G418 (500 μg/mL).

Virus: D-type HBV was concentrated from HepG2.2.15 cell culture supernatants.

Reagents: Main reagents used in this study included: FastStart Universal Probe Master (Roche), DNA extraction kit (Qiagen), 96-well plate (Costar), HBsAg ELISA kit (Autobio) and HBeAg ELISA kit (Autobio).

Instruments: The main instruments used in this study were real-time fluorescence quantitative PCR instrument (Thermo, QuantStudio™ 6 Flex) and multi-functional microplate reader (BioTek, Synergy2).

Test compound: WWS0 and ETV.

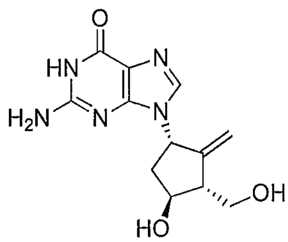

**ETV** ,

ETV was a drug that had been marketed for the treatment of HBV.

### 2. Experimental method:

2.1 On day 0, HepG2-NTCP cells were seeded into a 48-well cell culture plate at a density of 75,000 cells/well, and then the cells were cultured at 5% $CO_2$ and 37°C overnight.

2.2 On day 2, HBV was added to infect HepG2-NTCP cells.

2.3 On day 3, WWS0 and ETV single drugs were administered at seven different concentrations, respectively, or WWS0 in combination with ETV was administered at 5 different concentrations in orthorhombic proportion, added to 48-well plates with each combination having three duplicate plates.

**Table 9 Individual administered concentration settings of the test compound**

| Test compound | Test concentration (nM) | | | | | | | Solvent |
|---|---|---|---|---|---|---|---|---|
| ETV | 20 | 4 | 0.8 | 0.16 | 0.032 | 0.0064 | 0.00128 | DMSO |

(continued)

| Test compound | Test concentration (nM) | | | | | | | Solvent |
|---|---|---|---|---|---|---|---|---|
| WWS0 | 100 | 20 | 4 | 0.8 | 0.16 | 0.032 | 0.0064 | DMSO |

**Table 10 Administered arrangement of WWS0 in combination with ETV**

| | 1 | 2 | 3 | 4 | 5 | 6 | 7 | 8 |
|---|---|---|---|---|---|---|---|---|
| A | a0;b5 | a5;b5 | a4;b5 | a3;b5 | a2;b5 | a1;b5 | DMSO | Uninf |
| B | a0;b4 | a5;b4 | a4;b4 | a3;b4 | a2;b4 | a1;b4 | DMSO | Uninf |
| C | a0;b3 | a5;b3 | a4;b3 | a3;b3 | a2;b3 | a1;b3 | DMSO | Uninf |
| D | a0;b2 | a5;b2 | a4;b2 | a3;b2 | a2;b2 | a1;b2 | DMSO | Uninf |
| E | a0;b1 | a5;b1 | a4;b1 | a3;b1 | a2;b1 | a1;b1 | DMSO | Uninf |
| F | DMSO | a5;b0 | a4;b0 | a3;b0 | a2;b0 | a1;b0 | DMSO | Uninf |
| a=WWS0, b=ETV; 0 means no compound, and 1-5 means five concentrations. | | | | | | | | |

**Table 11 Administered concentration settings of WWS0 in combination with ETV**

| Test compound | | Concentration (nM) | | | | |
|---|---|---|---|---|---|---|
| | | 5 | 4 | 3 | 2 | 1 |
| a | WWS0 | 12 | 6 | 3 | 1.5 | 0.75 |
| b | ETV | 0.2 | 0.1 | 0.05 | 0.025 | 0.0125 |

2.4 On day 6, the cell supernatant was discarded and fresh medium containing the compounds was added, and the cells were cultured at 5% $CO_2$ and 37°C for 3 days.

2.5 On day 9, cell plate supernatant treated by compound was collected and DNA was extracted according to the QIAamp 96 DNA Blood Kit (12) instructions. HBeAg and HBsAg were detected according to the instructions of ELISA detection kit, meanwhile, the cytotoxicity of compound pairs was detected by CellTiter-Glo kit, and the chemiluminescence intensity (RLU) of each cell well was detected by multifunctional microplate reader according to the method of kit instructions. HBV DNA was quantified by qPCR method. HBV plasmid DNA was used as the standard, and standard HBV plasmid DNA concentration was diluted 7 points starting from $10^7$ copies/μL at a 10-fold gradient. The HBV DNA copy number was calculated in each test sample by fitting the standard curve using the HBV DNA copy number and CT values of each standard.

2.6 MacSynegy software (Prichard et al., 1990) was used to process the test data and analyze the effect parameters of combined administration of compound. Description of combined administration index:

A positive index is synergism, while a negative index is antagonism; and the absolute value of index < 25, that is, additive effect; the absolute value of the index is in the range of 25 to 50, that is, a mild but definite synergism or antagonism; the absolute value of the index is in the range of 50 to 100, that is, a moderate synergism or antagonism, which may be of great significance to the *in vivo* effect. The absolute value of the index is in the range of >100, that is, a high degree of synergism or antagonism, which may be of great significance to the *in vivo* effect.

**3. Experimental results:** See Table 12 and Table 13.

**Table 12 Anti-HBV activity and cytotoxicity of single drug**

| Test compound | HBeAg $EC_{50}$ (nM) | HBsAg $EC_{50}$ (nM) | HBV DNA $EC_{50}$ (nM) | Cytotoxicity $CC_{50}$ (nM) |
|---|---|---|---|---|
| ETV | >20 | >20 | 0.06 | >20 |

(continued)

| Test compound | HBeAg $EC_{50}$ (nM) | HBsAg $EC_{50}$ (nM) | HBV DNA $EC_{50}$ (nM) | Cytotoxicity $CC_{50}$ (nM) |
|---|---|---|---|---|
| WWS0 | 2.51 | 3.19 | 1.01 | >100 |

**Table 13 Results of combined administration of WWS0 and ETV (95% confidence interval)**

| Index | Combination | Synergistic index | Antagonistic Index | Cytotoxicity | Result |
|---|---|---|---|---|---|
| HBeAg | WWS0 + ETV | 33.82 | -6.03 | none | Mild coordination |
| HBsAg | WWS0 + ETV | 136.04 | 0 | none | Strong coordination |

**4. Experimental conclusion:** In the *in vitro* model of HepG2-NTCP infected by HBV, the test compound WWS0 inhibited HBV DNA, HBeAg and HBsAg in a dose-dependent manner; the combination of WWS0 and ETV shows that HBeAg and HBsAg have synergistic or additive anti-HBV activities; in the test compound WWWS0 single drug and combined action experiments, no obvious cytotoxicity is displayed within the test concentration range.

**Embodiment 3**

According to the method of embodiment 1, the test compound WWS0

(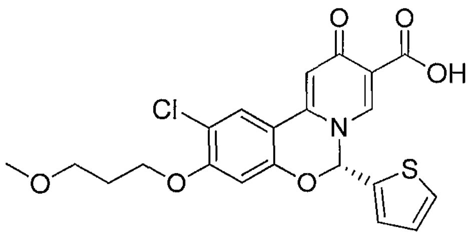)

was replaced by the compounds WWS01

(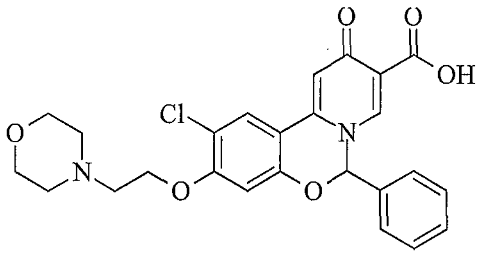),

WWS02

(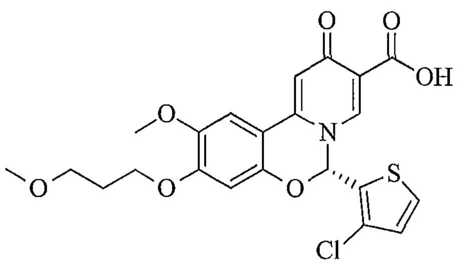),

and WWS03

( 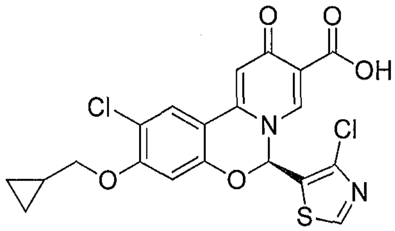 ),

the results showed that the two-drug combination of the test compounds WWS01, WWS02, WWS03 with Peg-IFNα-2a had a synergistic or additive effects on the inhibition of HBsAg and HBeAg, in which the synergistic index was in the range of 20.00 to 50.00 at the 95% confidence interval, and the antagonistic index was 0.

**Embodiment 4**

According to the method of embodiment 2, ETV (Entecavir) was replaced by TDF (tenofovir disoproxil fumarate) to obtain a combined administration result of the test compound WWS0 in a HepG2-NTCP *in vitro* infection HBV model: The combination of WWS0 with TDF had synergism on the inhibition of HBsAg and HBeAg, with the synergistic indexes being 86.55 and 25.49 respectively in the 95% confidence interval and the antagonistic indexes being 0 and 1.06.

**Embodiment 5**

According to the method of embodiment 1, interferon Peg-IFNα-2a was replaced by IFNα-2b, and the results of the two drug-combination of the test compound WWS0 and IFNα-2b and three drug-combination of WWS0 and TAF and IFNα-2b were obtained. The results showed that the synergistic indexes of both the two combinations for HBsAg and HBeAg were greater than 25, and had a synergism on the inhibition of HBsAg and HBeAg.

## Claims

**1.** A combination, comprising a compound represented by formula (I) or a pharmaceutically acceptable salt thereof,

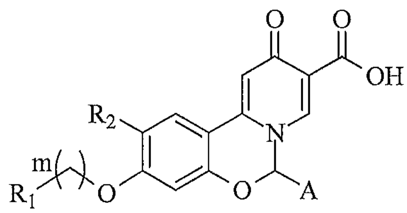

(I)

wherein,

$R_1$ is selected from H, OH, CN, $NH_2$, $C_{1-5}$ alkyl, $C_{1-5}$ heteroalkyl, $C_{2-5}$ alkynyl, $C_{3-6}$ cycloalkyl and 3- to 6-membered heterocycloalkyl, and the $C_{1-5}$ alkyl, $C_{1-5}$ heteroalkyl, $C_{2-5}$ alkynyl, $C_{3-6}$ cycloalkyl and 3- to 6-membered heterocycloalkyl are optionally substituted by 1, 2 or 3 R;
$R_2$ is selected from H, halogen, $C_{1-3}$ alkyl and $C_{1-3}$ heteroalkyl, and the $C_{1-3}$ alkyl and $C_{1-3}$ heteroalkyl are optionally substituted by 1, 2 or 3 R;
m is selected from 0, 1, 2, 3, 4 and 5;
A is selected from phenyl and 5- to 6-membered heteroaryl, and the phenyl and 5- to 6-membered heteroaryl are optionally substituted by 1, 2 or 3 R;
R is selected from H, halogen, OH, CN, $NH_2$, =O, $CH_3$, $CH_3CH_2$, $CH_3O$, $CF_3$, $CHF_2$, $CH_2F$;
the $C_{1-5}$ heteroalkyl, 3- to 6-membered heterocycloalkyl, $C_{1-3}$ heteroalkyl and 5- to 6-membered heteroaryl each independently comprise 1, 2 or 3 heteroatoms or heteroatom groups each independently selected from N, -O-, =O, -S-, -NH-, -(C=O)-, -(S=O)- and $-(S=O)_2-$,
wherein, the combination further comprises an immunomodulator.

**2.** The combination according to claim 1, wherein, the immunomodulator is selected from interferon, preferably peginterferon α-2a or interferon α-2b.

**3.** The combination according to claim 1, wherein, the combination further comprises a nucleotide reverse transcriptase inhibitor.

**4.** The combination according to claim 3, wherein, the nucleotide reverse transcriptase inhibitor is selected from tenofovir disoproxil fumarate and tenofovir alafenamide fumarate.

**5.** The combination according to claim 4, wherein, the immunomodulator is selected from interferon, preferably peginterferon α-2a or interferon α-2b.

**6.** A combination, comprising a compound represented by formula (I) or a pharmaceutically acceptable salt thereof, wherein the combination further comprises a nucleotide reverse transcriptase inhibitor or a nucleoside reverse transcriptase inhibitor.

**7.** The combination according to claim 6, wherein, the nucleotide reverse transcriptase inhibitor is selected from tenofovir disoproxil fumarate and tenofovir alafenamide fumarate.

**8.** The combination according to claim 6, wherein, the nucleoside reverse transcriptase inhibitor is selected from Entecavir.

**9.** The combination according to any one of claims 1-8, wherein, R is selected from H, F, Cl, Br, OH, $CH_3$, $CH_3O$, $CF_3$, $CHF_2$, $CH_2F$.

**10.** The combination according to any one of claims 1-8, wherein, $R_1$ is selected from H, OH, CN, $NH_2$, $CH_3$, $CH_3CH_2$, $CH_3CH_2CH_2$, $CH_3CH_2CH_2CH_2$, $CH_3O$, $CH_3CH_2O$, $CH_3S$, $CH_3S(=O)$, $CH_3S(=O)_2$, $CH_3SCH_2$, $CH_3CH_2S$, $CH_3NH$,

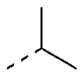, 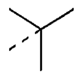, 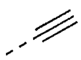, 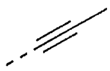,

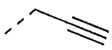, 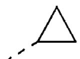, 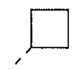, 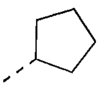, 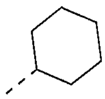,

pyrrolidinyl, piperidinyl, tetrahydropyranyl, morpholinyl, 2-pyrrolidonyl and 3-pyrrolidonyl, and the $CH_3$, $CH_3CH_2$, $CH_3CH_2CH_2$, $CH_3CH_2CH_2CH_2$, $CH_3O$, $CH_3CH_2O$, $CH_3S$, $CH_3S(=O)$, $CH_3S(=O)_2$, $CH_3SCH_2$, $CH_3CH_2S$, $CH_3NH$,

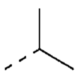, 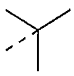, 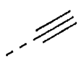, 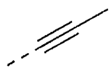, 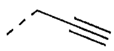, 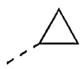, 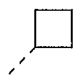, 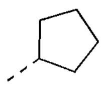, 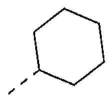,

pyrrolidinyl, piperidinyl, tetrahydropyranyl, morpholinyl, 2-pyrrolidonyl and 3-pyrrolidonyl are optionally substituted by 1, 2 or 3 R.

**11.** The combination according to claim 10, wherein, $R_1$ is selected from H, OH, CN, $NH_2$, $CH_3$, $CH_3CH_2$, $CH_3CH_2CH_2$, $CH_3CH_2CH_2CH_2$, $CH_3O$, $CH_3CH_2O$, $CH_3S$, $CH_3S(=O)$, $CH_3S(=O)_2$, $CH_3SCH_2$, $CH_3CH_2S$, $CH_3NH$,

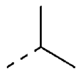, 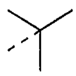, 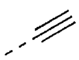, 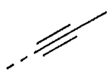, 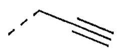,

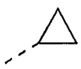, 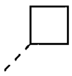, 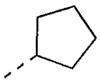, 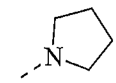, 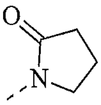, 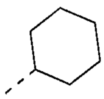, 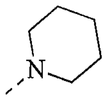, 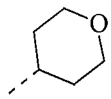 and 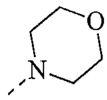,

and the $CH_3$, $CH_3CH_2$, $CH_3CH_2CH_2$, $CH_3CH_2CH_2CH_2$, $CH_3O$, $CH_3CH_2O$, $CH_3S$, $CH_3S(=O)$, $CH_3S(=O)_2$, $CH_3SCH_2$, $CH_3CH_2S$, $CH_3NH$,

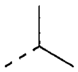, 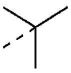, 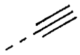, 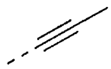, 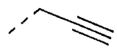,

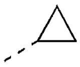, 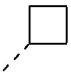, 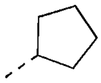, 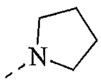, 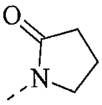, 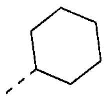, 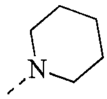, 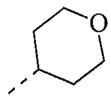 and 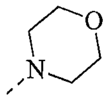

are optionally substituted by 1, 2 or 3 R.

**12.** The combination according to claim 11, wherein, $R_1$ is selected from H, OH, $CH_3$, $CHF_2$, $CH_3O$,

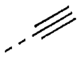, 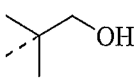, 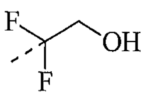, 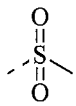, 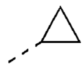, 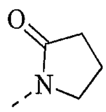 and 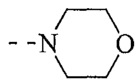.

**13.** The combination according to any one of claims 1-8, wherein, $R_2$ is selected from H, F, Cl, Br, $CH_3$, $CH_3CH_2$, $CH_3O$, $CH_3CH_2O$ and

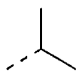,

and the $CH_3$, $CH_3CH_2$, $CH_3O$, $CH_3CH_2O$ and

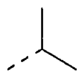

are optionally substituted by 1, 2 or 3 R.

**14.** The combination according to claim 13, wherein, $R_2$ is selected from Cl and $CH_3O$.

**15.** The combination according to any one of claims 1-8, wherein, A is selected from: phenyl, thienyl, thiazolyl, isothiazolyl, oxazolyl and isoxazolyl, each of which is optionally substituted by 1, 2 or 3 R.

**16.** The combination according to claim 15, wherein, A is selected from:

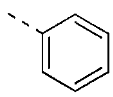,

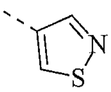, 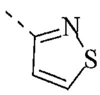, 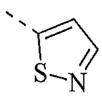, 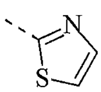, 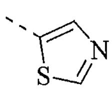, 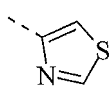, 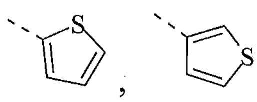

and

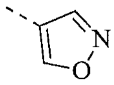,

each of which is optionally substituted by 1, 2 or 3 R.

**17.** The combination according to claim 16, wherein, A is selected from

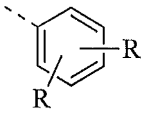,

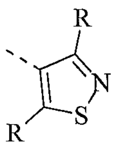, 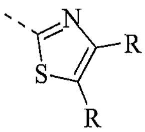, 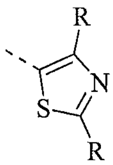, 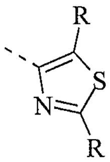, 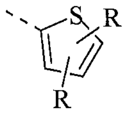, 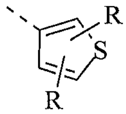 and 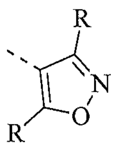.

**18.** The combination according to claim 17, wherein, A is selected from

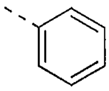,

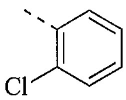, 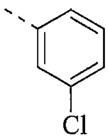, 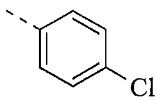, 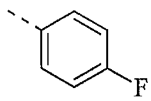, 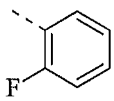, 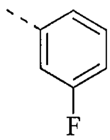, 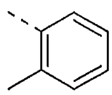, 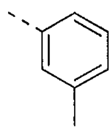,

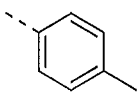, 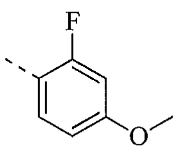, 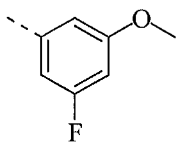, 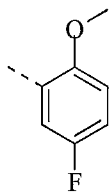, 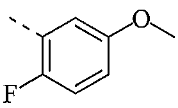, 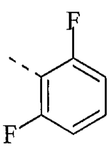, 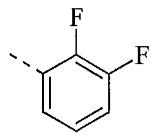,

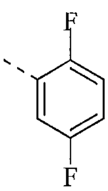, 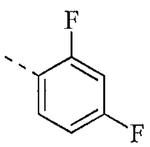, 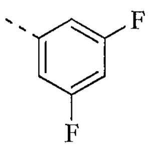, 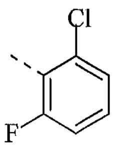, 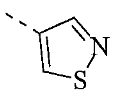, 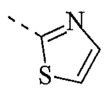, 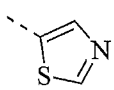, 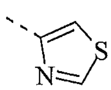,

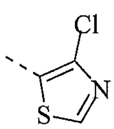, 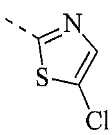, 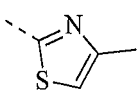, 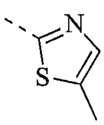, 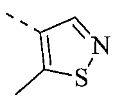, 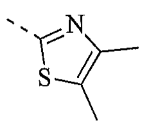, 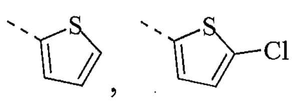,

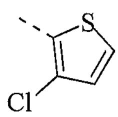, 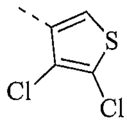 and 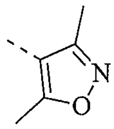.

**19.** The combination according to any one of claims 1 to 8, wherein, m is 3.

**20.** The combination according to claim 19, wherein, $R_2$ is selected from Cl and $CH_3O$.

**21.** The combination according to claim 20, wherein, $R_1$ is $CH_3O$.

**22.** The combination according to any one of claims 1 to 8, wherein, m is 1.

**23.** The combination according to claim 22, wherein, $R_2$ is Cl.

**24.** The combination according to claim 23, wherein, $R_1$ is

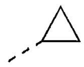.

**25.** The combination according to claim 21 or 24, wherein, A is selected from:

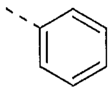,

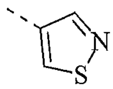, 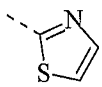, 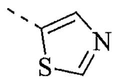, 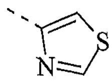, 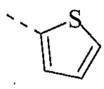 and 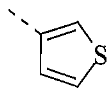,

each of which is optionally substituted by 1, 2 or 3 R.

**26.** The combination according to any one of claims 1 to 8, wherein, the compound is selected from:

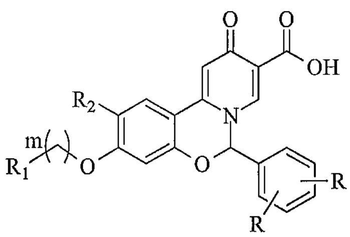

(II),

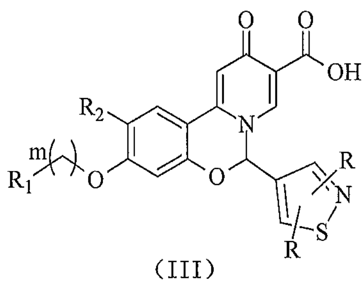

(III),

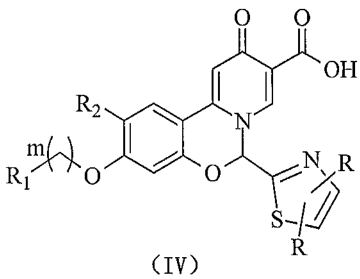

(IV),

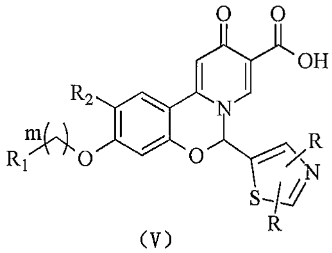
(V)
,
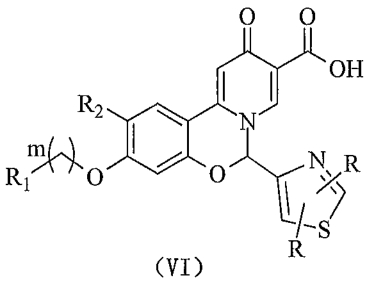
(VI)
,
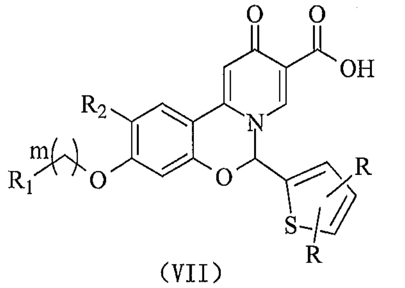
(VII)
,

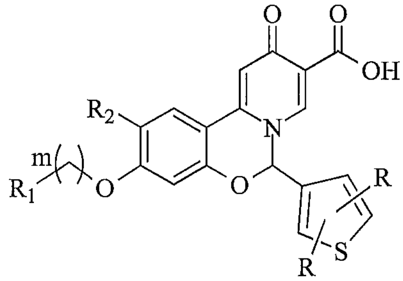
(VIII)
,
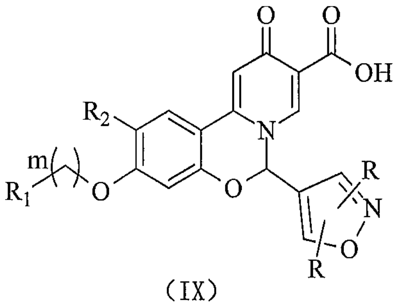
(IX)
,

wherein,
$R_1$, $R_2$, R and m are as defined in any one of claims 1 to 8.

**27.** The combination according to any one of claims 1 to 8, wherein, the compound is selected from:

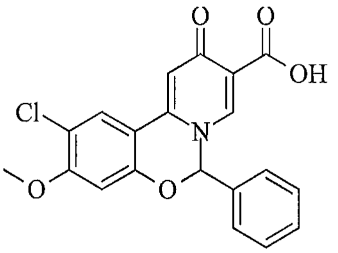
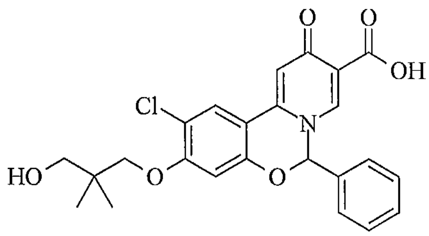

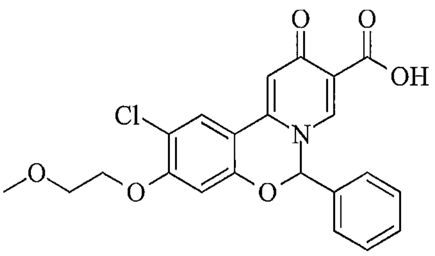
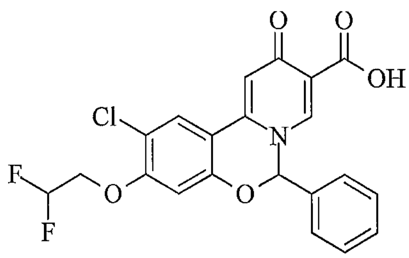

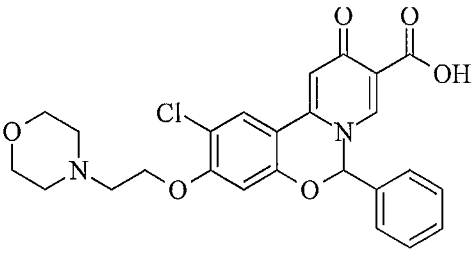
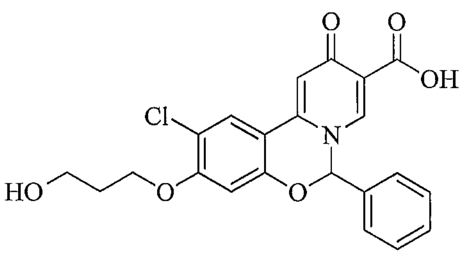

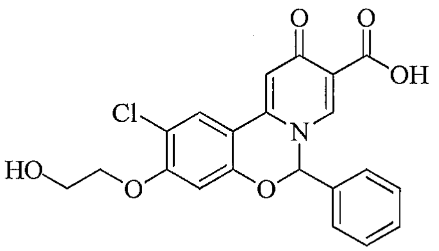

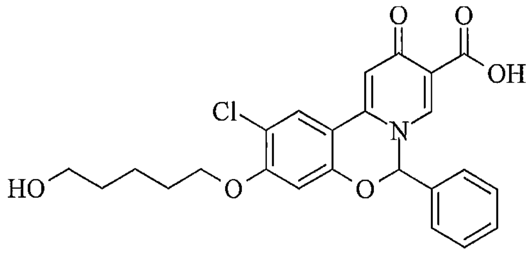

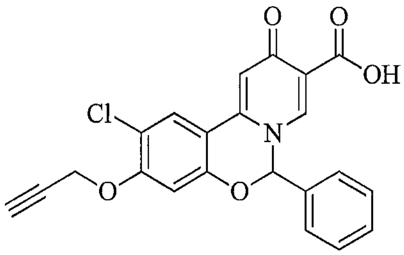

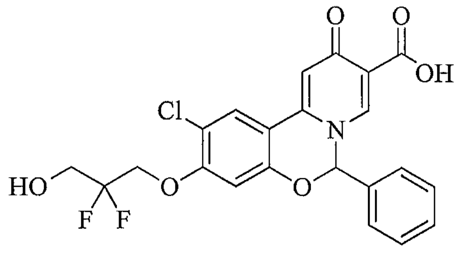

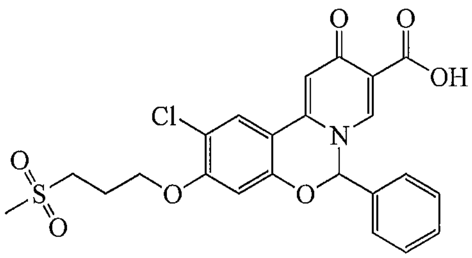

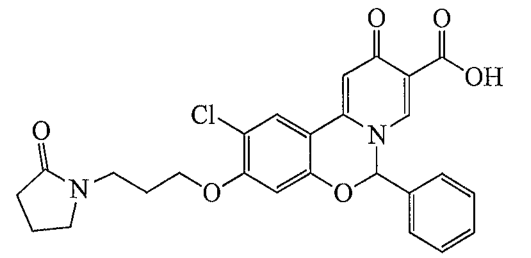

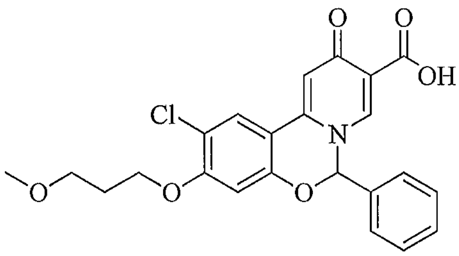

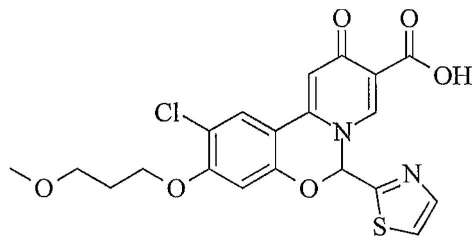

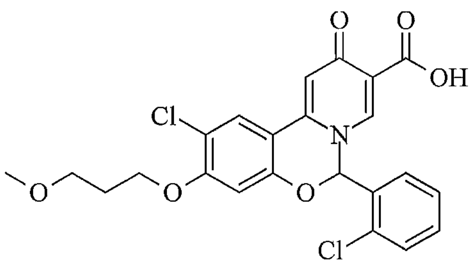

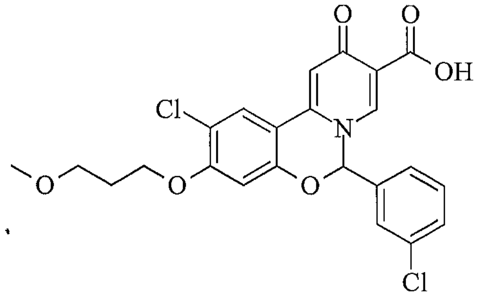

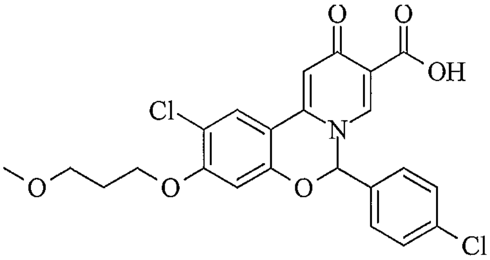

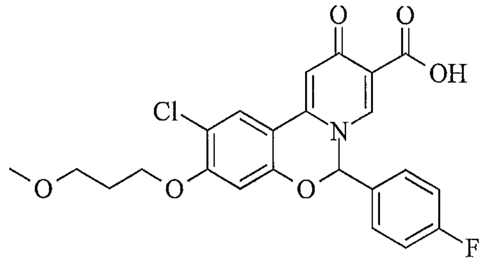

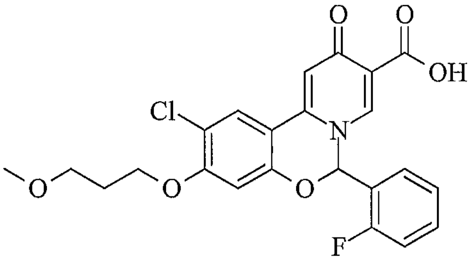

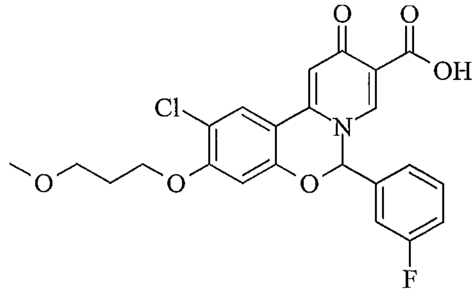

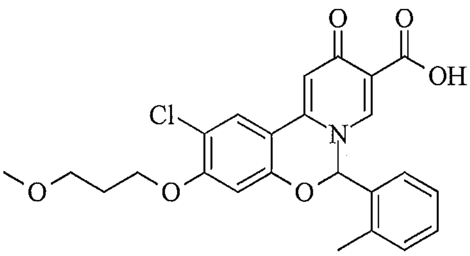

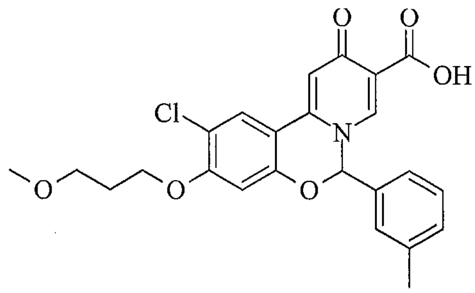

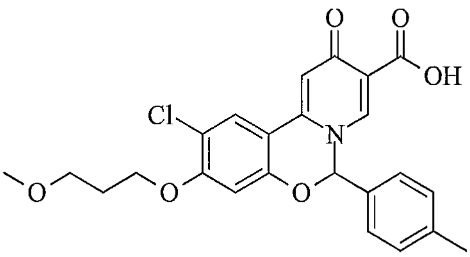

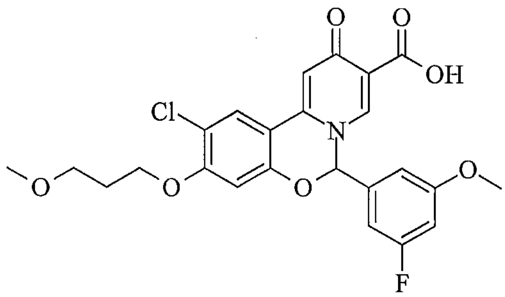

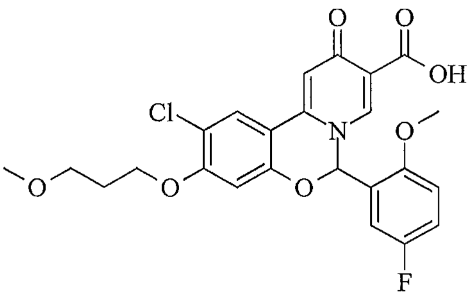

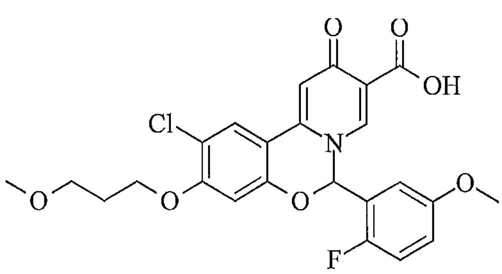

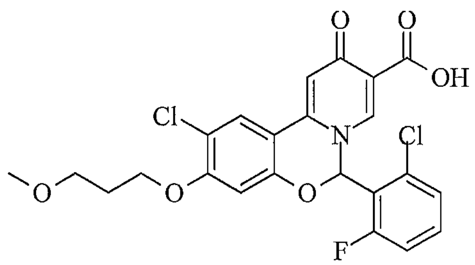

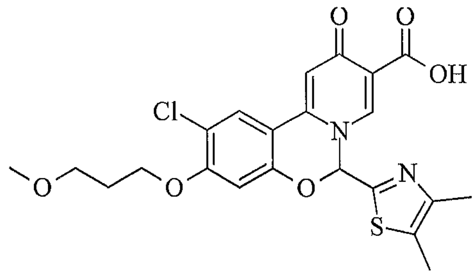

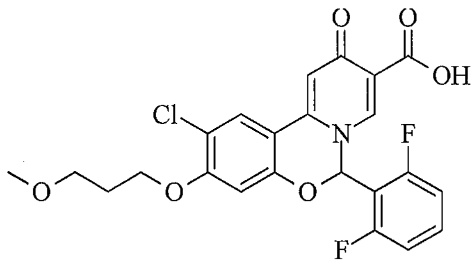

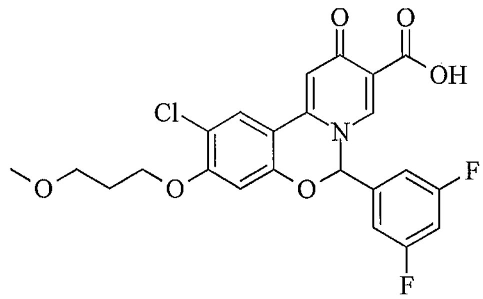

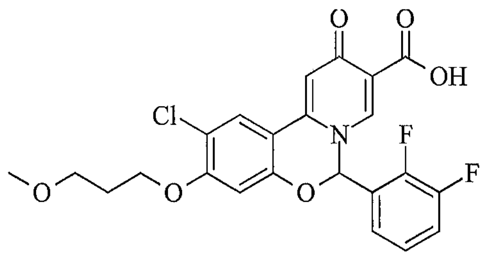

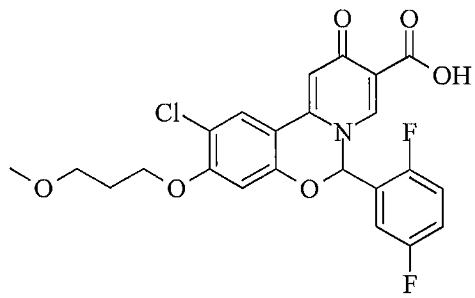

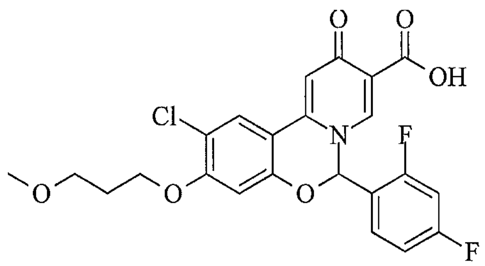

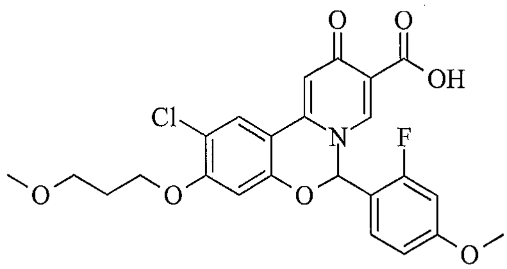

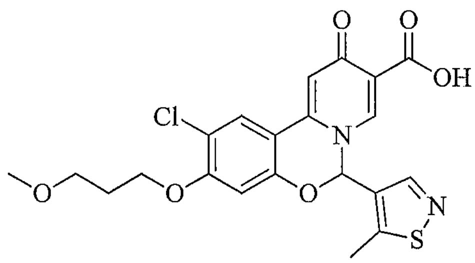

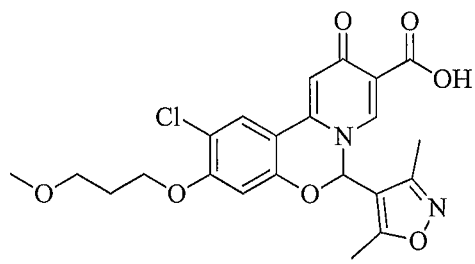

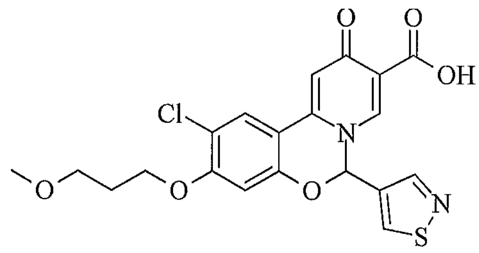

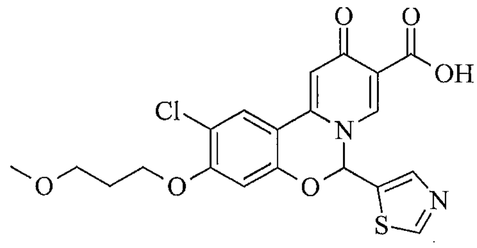

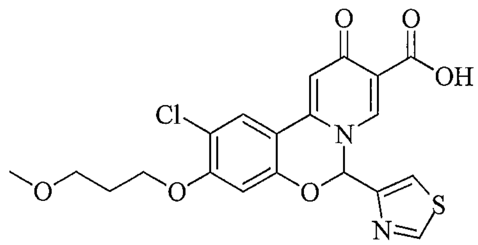

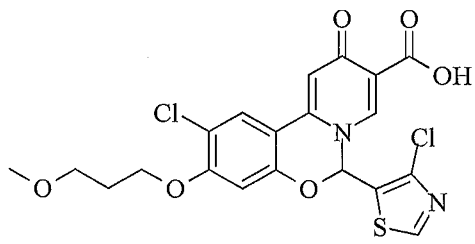

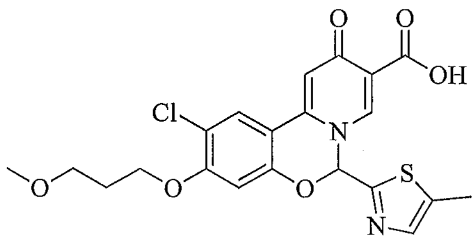

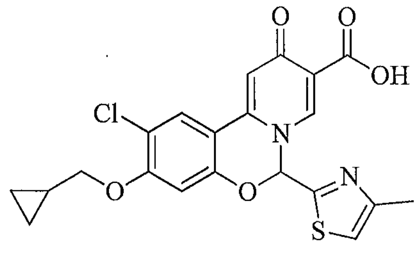

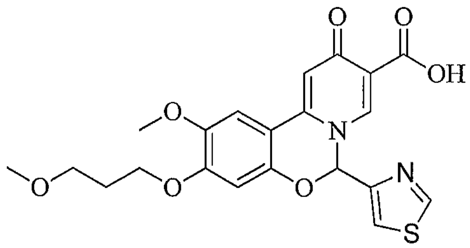

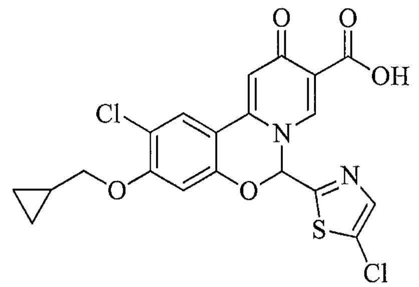

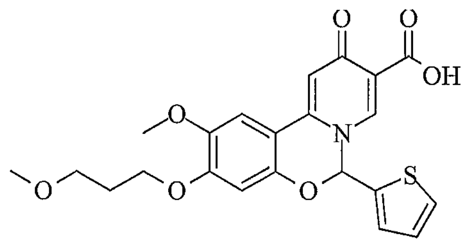

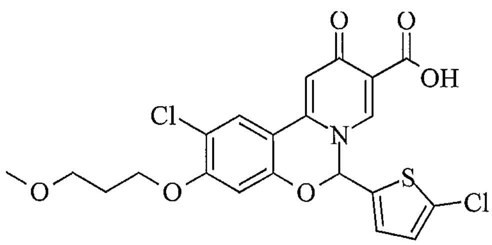

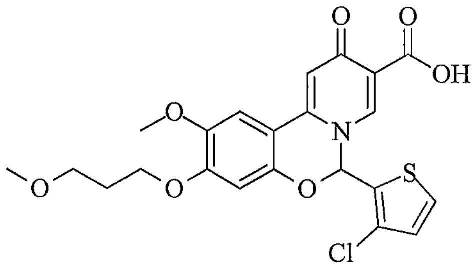

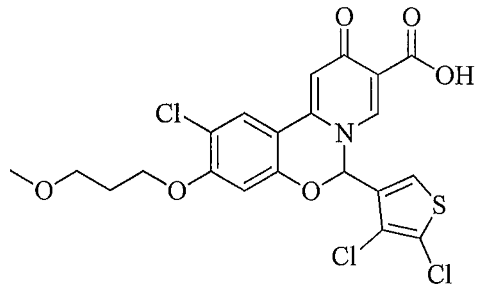

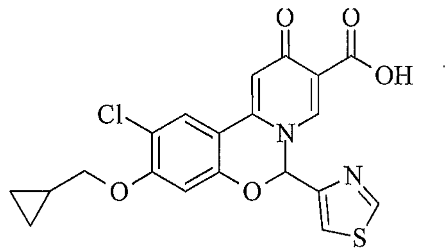

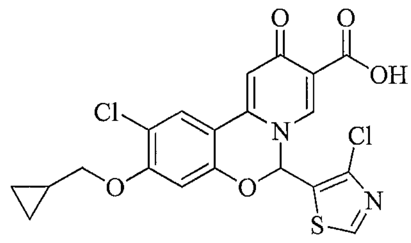

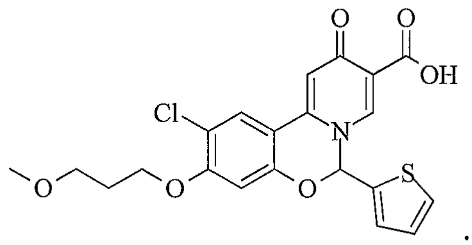

**28.** The combination according to claim 27, wherein, the compound is selected from:

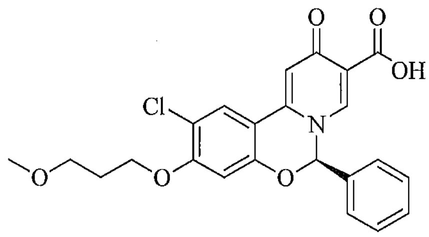

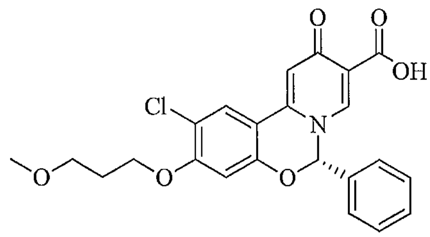

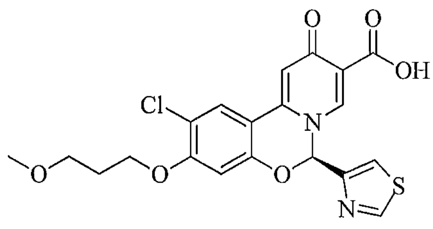

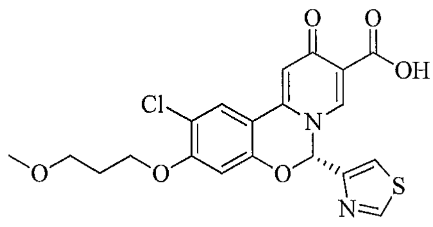

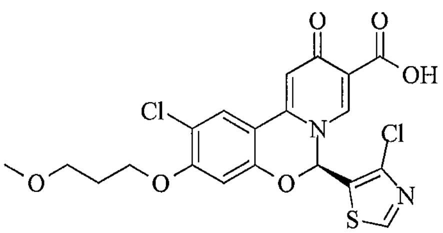

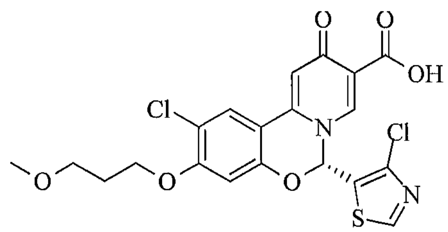

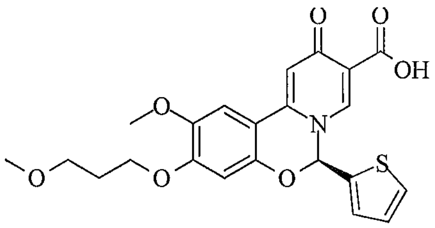

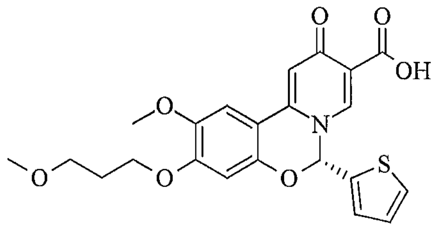

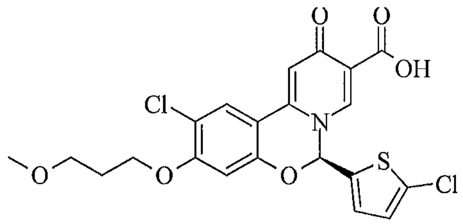

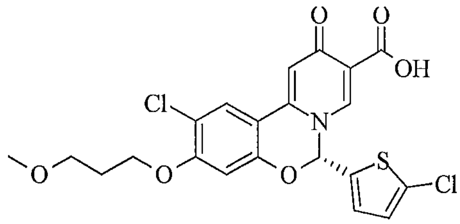

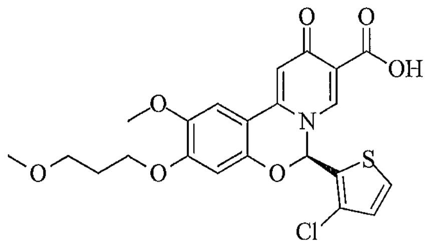

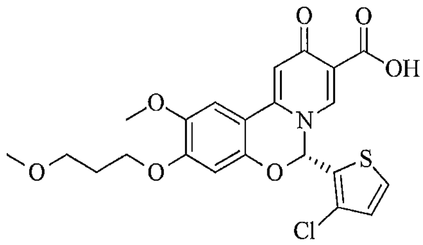

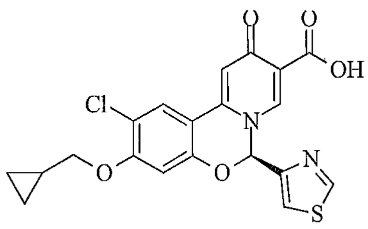

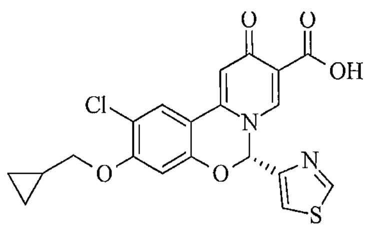

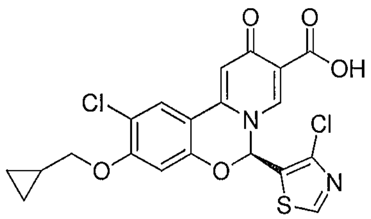

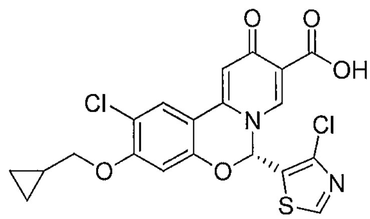

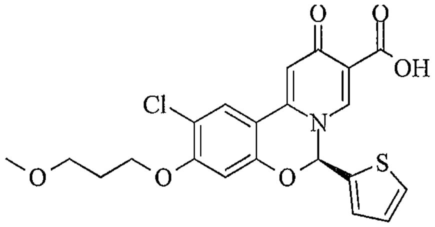

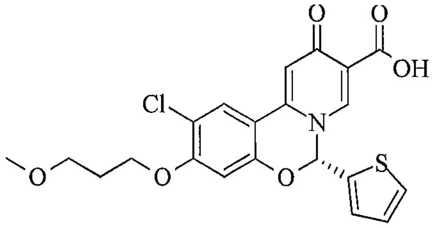

.

**29.** Use of the combination according to any one of claims 1 to 28 in the manufacture of a medicament for the treatment of hepatitis B.

**30.** A composition, comprising the combination according to any one of claims 1 to 28 and at least one pharmaceutically acceptable carrier and/or excipient.

**31.** A kit, comprising the combination according to any one of claims 1 to 28 or the composition according to claim 29.

**32.** Use of the composition according to claim 30 or the kit according to claim 31 in the manufacture of a medicament for the treatment of hepatitis B.

## INTERNATIONAL SEARCH REPORT

International application No.
**PCT/CN2021/093805**

**A. CLASSIFICATION OF SUBJECT MATTER**

A61K 31/5365(2006.01)i; A61K 38/21(2006.01)i; A61K 31/52(2006.01)i; A61K 31/522(2006.01)i; A61P 31/20(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

DWPI, SIPOABS, CNABS, CNTXT, TWTXT, WOTXT, EPTXT, USTXT, CNKI(CN), REGISTRY, CAPLUS, MEDLINE: 干扰素, 恩替卡韦, 替诺福韦, 乙肝, 式I结构检索, Interferon, Entecavir, Tenofovir, hepatitis B, structure search based on formula I,

**C. DOCUMENTS CONSIDERED TO BE RELEVANT**

| Category* | Citation of document, with indication, where appropriate, of the relevant passages | Relevant to claim No. |
|---|---|---|
| X | WO 2018161960 A1 (FUJIAN COSUNTER PHARMACEUTICAL CO., LTD. et al.) 13 September 2018 (2018-09-13)<br>claims 1-23, description page 2 paragraph 2 | 1-32 |
| X | WO 2019169539 A1 (PHARMARESOURCES SHANGHAI CO., LTD.) 12 September 2019 (2019-09-12)<br>abstract, description page 49 paragraph [0121], page 57 embodiments 6-7 | 1-32 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 July 2021** | **11 August 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/093805**

| Patent document cited in search report | Publication date (day/month/year) | Patent family member(s) | Publication date (day/month/year) |
|---|---|---|---|
| WO 2018161960 A1 | 13 September 2018 | CA 3055442 C | 23 March 2021 |
| | | MX 2019010736 A | 05 November 2019 |
| | | KR 102087397 B1 | 11 March 2020 |
| | | CN 109071564 A | 21 December 2018 |
| | | CN 109071564 B | 27 August 2019 |
| | | AU 2018232071 A1 | 17 October 2019 |
| | | SG 11201908101 Y A | 30 October 2019 |
| | | JP 6783424 B2 | 11 November 2020 |
| | | JP 2020509075 A | 26 March 2020 |
| | | EP 3590942 A4 | 08 January 2020 |
| | | CA 3055442 A1 | 13 September 2018 |
| | | US 2020039995 A1 | 06 February 2020 |
| | | IL 269142 A | 30 July 2020 |
| | | PH 12019502052 A1 | 06 July 2020 |
| | | EP 3590942 A1 | 08 January 2020 |
| | | EA 201992082 A1 | 12 February 2020 |
| | | BR 112019018650 A2 | 07 April 2020 |
| | | KR 20190117799 A | 16 October 2019 |
| WO 2019169539 A1 | 12 September 2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010412771
- WO 2016128335 A1


### Non-patent literature cited in the description


- **BERGE et al.** Pharmaceutical Salts. *Journal of Pharmaceutical Science,* 1977, vol. 66, 1-19
- Remington: The Science and Practice of Pharmacy. Lippincott, Williams & Wilkins, 2005